(19) Europäisches Patentamt
European Patent Office
Office européen des brevets

(11) Veröffentlichungsnummer : **0 331 966 B1**

(12) # EUROPÄISCHE PATENTSCHRIFT

(45) Veröffentlichungstag der Patentschrift : **23.09.92 Patentblatt 92/39**

(21) Anmeldenummer : **89102967.0**

(22) Anmeldetag : **21.02.89**

(51) Int. Cl.$^5$ : **C07D 277/42,** C07D 277/36, C07D 277/34, C07D 263/48, C07D 263/46, A01N 43/78, A01N 43/76

(54) **Substituierte Acrylsäureester.**

(30) Priorität : **05.03.88 DE 3807232**

(43) Veröffentlichungstag der Anmeldung : **13.09.89 Patentblatt 89/37**

(45) Bekanntmachung des Hinweises auf die Patenterteilung : **23.09.92 Patentblatt 92/39**

(84) Benannte Vertragsstaaten : **BE CH DE ES FR GB IT LI NL**

(56) Entgegenhaltungen :
**EP-A- 0 006 368**
**EP-A- 0 061 425**
**DE-A- 2 129 012**
**CHEMICAL ABSTRACTS, Band 106, Nr. 7, 16. Februar 1987, Columbus, Ohio, US; BARTON, D.H.R. et al.: "The invention of newradical chain reactions. Part 9. Further radical chemistry of thiohydroxamic esters; formation of carbon-carbon bonds." Seite 638,Spalte 2, Zusammenfassung-Nr. 50 146e**
**CHEMICAL ABSTRACTS, Band 101, Nr. 9, 27. August 1984, Columbus, Ohio, US; BARTON, D.H.R. et al.: "Formation of carbon-carbon-bonds with radicals derived from the esters of thiohydroxamic acids" Seite 637, Spalte 2, Zusammenfassung-Nr. 72 652r**

(56) Entgegenhaltungen :
**CHEMICAL ABSTRACTS, Band 106, Nr. 11, 16. März 1987, Columbus, Ohio, US; BASF AG: "Fungicidal acrylates." Seite 228, Spalte2, Zusammenfassung-Nr. 80 390x**
**CHEMICAL ABSTRACTS, Band 106, Nr. 13, 30. März 1987, Columbus, Ohio, US; IMPERIAL CHEMICAL INDUSTRIES PLC.: "Heterocycliccompounds as fungicides" Seite 270, Spalte 1, Zusammenfassung-Nr. 98 100n**

(73) Patentinhaber : **BAYER AG**
**W-5090 Leverkusen 1 Bayerwerk (DE)**

(72) Erfinder : **Kleefeld, Gerg, Dr.**
**Otto-Hahn-Strasse 87**
**W-4000 Düsseldorf 13 (DE)**
Erfinder : **Klausener, Alexander, Dr.**
**Bendenstrasse 16**
**W-5190 Stolberg (DE)**
Erfinder : **Krämer, Wolfgang, Dr.**
**Rosenkranz 25**
**W-5093 Burscheid 2 (DE)**
Erfinder : **Brandes, Wilhelm, Dr.**
**Eichendorffstrasse 3**
**W-5653 Leichlingen 1 (DE)**
Erfinder : **Dutzmann, Stefan, Dr.**
**Leinenweberweg 33**
**W-4000 Düsseldorf 13 (DE)**
Erfinder : **Hänssler, Gerd, Dr.**
**Am Arenzberg 58a**
**W-5090 Leverkusen 3 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue substituierte Acrylsäureester, mehrere Verfahren zu ihrer Herstellung und ihre Verwendung in Schädlingsbekämpfungsmitteln.

Es ist bekannt, daß bestimmte substituierte Acrylsäureester, wie beispielsweise die Verbindung 2-(2-Benzoylpyrrol-1-yl)-3-methoxy-acrylsäuremethylester oder die Verbindung 3-Methoxy-2-(2-methylphenyl)-acrylsäuremethylester oder die Verbindung 2-(2-Benzyloxyphenyl)-3-methoxy-acrylsäurephenylester fungizide Eigenschaften besitzen (vgl. z.B. EP 206 523; EP 178 816; DE-OS 35 19 282).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Acrylsäureester der allgemeinen Formel (I),

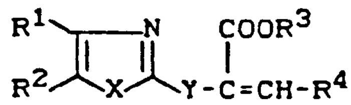

(I)

in welcher

$R^1$ und $R^2$ unabhängig voneinander jeweils für Wasserstoff, für geradkettiges oder verzweigtes Alkyl mit 1 bis 8 Kohlenstoffatomen, für geradkettiges oder verzweigtes Alkenyl mit 2 bis 8 Kohlenstoffatomen, für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder Aryl mit 6 bis 10 Kohlenstoffatomen im jeweiligen Arylteil stehen, wobei als Arylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;

außerdem für einen gegebenenfalls einfach oder mehrfach, gleich oder verschieden substituierten Heteroarylrest mit 2 bis 9 Kohlenstoffatomen und 1 bis 4 gleichen oder verschiedenen Heteroatomen - insbesondere Stickstoff, Sauerstoff und/oder Schwefel- stehen, wobei als Substituenten die oben genannten Arylsubstituenten infrage kommen,

$R^3$ für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ und $R^2$ gennanten infrage kommen,

$R^4$ für Dialkylamino mit jeweils 1 bis 6 Kohlenstoffatomen in den einzelnen Alkylteilen steht oder für einen Rest -Z-$R^5$ steht,

X für Sauerstoff oder Schwefel steht und

Y für Sauerstoff, Schwefel oder für einen Rest

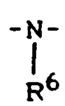

steht,
wobei

$R^5$ für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ und $R^2$ genannten infrage kommen,

$R^6$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil oder Aryl mit jeweils 6 bis 10 Kohlenstoffatomen im jeweiligen Arylteil steht, wobei als Substituenten im Arylteil jeweils die bei $R^1$ und $R^2$ gennanten infrage kommen und

Z für Sauerstoff oder Schwefel steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Acrylsäureester der allgemeinen Formel (I),

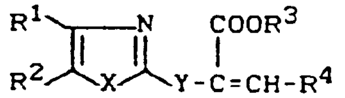

(I)

in welcher

$R^1$, $R^2$, $R^3$, $R^4$ X und Y die oben angegebene Bedeutung haben,

nach einem der um Folgenden beschriebenen Verfahren erhält:

(a) Man erhält substituierte Acrylsäureester der Formel (Ia),

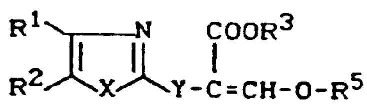

(Ia)

in welcher

$R^1$, $R^2$, $R^3$, $R^5$, X und Y die oben angegebene Bedeutung haben,

wenn man Hydroxyacrylsäureester oder deren Alkalimetallsalze der Formel (II),

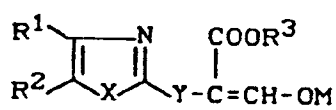

(II)

in welcher

M für Wasserstoff oder für ein Alkalimetallkation steht und

$R^1$, $R^2$, $R^3$, X und Y die oben angegebene Bedeutung haben,

mit Alkylierungsmitteln der Formel (III),

$$R^5\text{-}E^1 \qquad (III)$$

in welcher

$E^1$ für eine elektronenanziehende Abgangsgruppe steht und

$R^5$ die oben angegebene Bedeutung hat,

gegebenenfalls in Gegenwart eines Verdünnungsmittels und gegebenenfalls in Gegenwart eines Reaktionshilfsmittels umsetzt;

(b) man erhält substituierte Acrylsäureester der Formel (Ib),

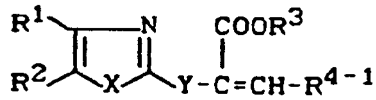
(Ib)

in welcher

$R^{4-1}$ für Dialkylamino steht und

$R^1$, $R^2$, $R^3$, X und Y die oben angegebene Bedeutung haben,

wenn man substituierte Essigsäureester der Formel (IV),

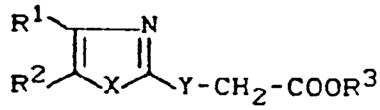
(IV)

in welcher

$R^1$, $R^2$, $R^3$, X und Y die oben angegebene Bedeutung haben,

mit Formamiden der Formel (Va),

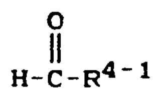
(Va)

in welcher

$R^{4-1}$ die oben angegebene Bedeutung hat,

oder mit deren Derivaten der Formel (Vb),

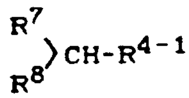
(Vb)

in welcher

$R^7$ und $R^8$ unabhängig voneinander jeweils für Alkoxy oder Dialkylamino stehen und

$R^{4-1}$ die oben angegebene Bedeutung hat,

gegebenenfalls in Gegenwart eines Verdünnungsmittels umsetzt;

(c) man erhält substituierte Acrylsäureester der Formel (Ic),

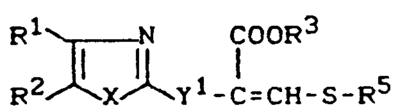
(Ic)

in welcher

$Y^1$ für Schwefel oder für einen Rest

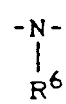

steht, und

$R^1$, $R^2$, $R^3$, $R^5$, $R^6$ und X die oben angegebene Bedeutung haben,

wenn man Oxalsäurederivate der Formel (VI),

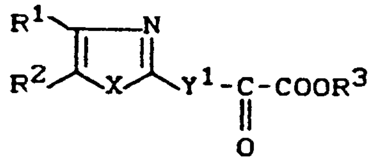

(VI)

in welcher

$R^1$, $R^2$, $R^3$, X und $Y^1$ die oben angegebene Bedeutung haben, mit metallorganischen Verbindungen der Formel (VII),

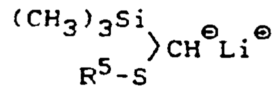

(VII)

in welcher

$R^5$ die oben angegebene Bedeutung hat, gegebenenfalls in Gegenwart eines Verdünnungsmittels umsetzt;

(d) man erhält substituierte Acrylsäureester der Formel (Id),

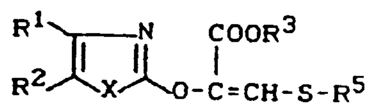

(Id)

in welcher

$R^1$, $R^2$, $R^3$, $R^5$ und X die oben angegebene Bedeutung haben, wenn man substituierte Acrylsäureester der Formel (VIII),

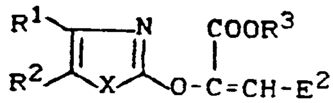

(VIII)

in welcher

$E^2$ für eine elektronenanziehende Abgangsgruppe steht und $R^1$, $R^2$, $R^3$ und X die oben angegebene Bedeutung haben, mit Thiolen der Formel (IX),

$R^5$-SH (IX)

in welcher

$R^5$ die oben angegebene Bedeutung hat, gegebenenfalls in Gegenwart eines Verdünnungsmittels und gegebenenfalls in Gegenwart eines Reaktionshilfsmittels umsetzt.

Schließlich wurde gefunden, daß die neuen substituierten Acrylsäureester der allgemeinen Formel (II) eine gute Wirkung gegen Schädlinge besitzen.

Überraschenderweise zeigen die erfindungsgemäßen substituierten Acrylsäureester der allgemeinen Formel (I) z.B. eine erheblich bessere fungizide Wirksamkeit als die aus dem Stand der Technik bekannten Acrylsäureester, wie beispielsweise die Verbindung 2-(2-Benzoylpyrrol-1-yl)-3-methoxyacrylsäuremethylester oder die Verbindung 3-Methoxy-2-(2-methylphenyl)-acrylsäuremethylester oder die Verbindung 2-(2-Benzyloxyphenyl)-3-methoxy-acrylsäurephenylester, welche chemisch und wirkungsmäßig naheliegende Verbindungen sind.

Die erfindungsgemäßen substituierten Acrylsäureester sind durch die Formel (I) allgemein definiert.

Besonders bevorzugt sind Verbindungen der Formel (I), bei welchen

$R^1$ und $R^2$ unabhängig voneinander jeweils für Wasserstoff, Methyl, Ethyl, n- oder i-Propyl, n-, i-, s- oder t-Butyl, für Allyl, n- oder i-Butenyl, für jeweils gegebenenfalls im Arylteil oder im Heteroarylteil ein- bis dreifach, gleich oder verschieden substituiertes Benzyl, Phenylethyl, Phenylethenyl, Phenyl, Naphthyl, Pyridyl, Thienyl oder Furyl stehen, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl,

Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen,

$R^4$ für Dialkylamino mit jeweils 1 bis 4 Kohlenstoffatomen in den einzelnen Alkylteilen steht oder für einen Rest -Z-$R^5$ steht,

X für Sauerstoff oder Schwefel steht und

Y für Sauerstoff, Schwefel oder für einen Rest

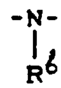

steht,

wobei

$R^5$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen;

$R^6$ für Wasserstoff, Methyl, Ethyl, n- oder i-Propyl, n- i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ und $R^2$ genannten infrage kommen und

Z für Sauerstoff oder Schwefel steht.

Ganz besonders bevorzugt sind Verbindungen der Formel (I), bei welchen

$R^1$ und $R^2$ unabhängig voneinander jeweils für Wasserstoff, Methyl, Ethyl, n- oder i-Propyl oder für jeweils gegebenenfalls ein- bis dreifach, gleich oder verschieden substituiertes Phenyl oder Naphthyl stehen, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy,

$R^3$ für Methyl, Ethyl oder Benzyl steht,

$R^4$ für Dimethylamino, Diethylamino oder für einen Rest -Z-$R^5$ steht,

X für Sauerstoff oder Schwefel steht und

Y für Sauerstoff, Schwefel oder für einen Rest

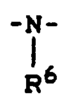

steht,

wobei

$R^5$ für Methyl, Ethyl, n- oder i-Propyl oder Benzyl steht,

$R^6$ für Wasserstoff, Methyl, Ethyl, Acetyl, Propionyl oder für jeweils gegebenenfalls einfach oder zweifach, gleich oder verschieden durch Fluor, Chlor, Brom, Methyl, Ethyl und/oder Trifluormethyl substituiertes Benzyl oder Phenyl steht und

Z für Sauerstoff oder Schwefel steht.

Insbesondere bevorzugt sind Verbindungen der Formel (I), bei welchen

$R^1$ für Wasserstoff, Methyl, Ethyl, n- oder i-Propyl oder für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Methoximinoethyl, Cyclopentyl, 1,3-Propandiyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Wasserstoff, Methyl, Ethyl, n- oder i-Propyl steht,

$R^3$ für Methyl oder Ethyl steht,
$R^4$ für Methoxy, Ethoxy, Methylthio oder Dimethylamino steht,
X für Sauerstoff oder Schwefel steht und
Y für einen N-Methyl-Rest steht.

Im einzelnen seien außer den bei den Herstellungsbeispielen genannten Verbindungen die folgenden substituierten Acrylsäureester der allgemeinen Formel (I) genannt:

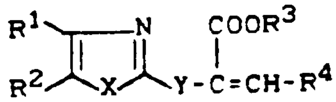

(I)

| $R^1$ | $R^2$ | $R^3$ | $R^4$ | X | Y |
|---|---|---|---|---|---|
| 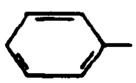 | H | $CH_3$ | $OCH_3$ | S | $N-CH_3$ |
| 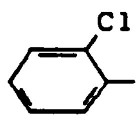 | H | $CH_3$ | $OCH_3$ | S | $N-CH_3$ |
| 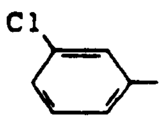 | H | $CH_3$ | $OCH_3$ | S | $N-CH_3$ |
| 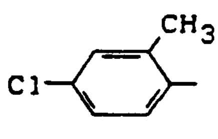 | H | $CH_3$ | $OCH_3$ | S | $N-CH_3$ |
| 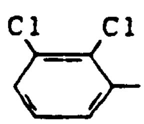 | H | $CH_3$ | $OCH_3$ | S | $N-CH_3$ |
| 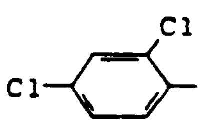 | H | $CH_3$ | $OCH_3$ | S | $N-CH_3$ |
| 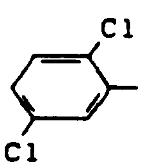 | H | $CH_3$ | $OCH_3$ | S | $N-CH_3$ |
| 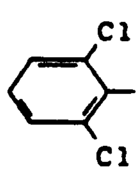 | H | $CH_3$ | $OCH_3$ | S | $N-CH_3$ |

| $R^1$ | $R^2$ | $R^3$ | $R^4$ | X | Y |
|---|---|---|---|---|---|
| 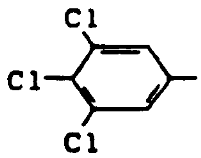 | H | $CH_3$ | $OCH_3$ | S | $N-CH_3$ |
| 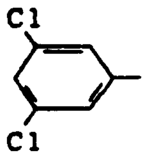 | H | $CH_3$ | $OCH_3$ | S | $N-CH_3$ |
| 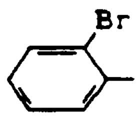 | H | $CH_3$ | $OCH_3$ | S | $N-CH_3$ |
| 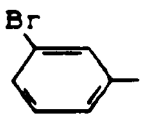 | H | $CH_3$ | $OCH_3$ | S | $N-CH_3$ |
| 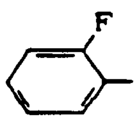 | H | $CH_3$ | $OCH_3$ | S | $N-CH_3$ |
| 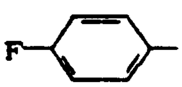 | H | $CH_3$ | $OCH_3$ | S | $N-CH_3$ |
| 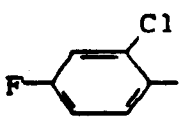 | H | $CH_3$ | $OCH_3$ | S | $N-CH_3$ |
| 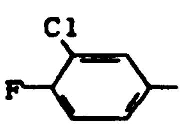 | H | $CH_3$ | $OCH_3$ | S | $N-CH_3$ |

| $R^1$ | $R^2$ | $R^3$ | $R^4$ | X | Y |
|---|---|---|---|---|---|
| 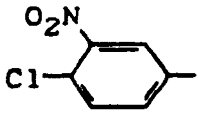 | H | $CH_3$ | $OCH_3$ | S | $N-CH_3$ |
| 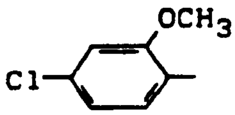 | H | $CH_3$ | $OCH_3$ | S | $N-CH_3$ |
| 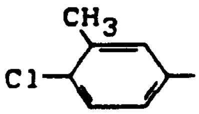 | H | $CH_3$ | $OCH_3$ | S | $N-CH_3$ |
| 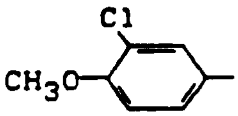 | H | $CH_3$ | $OCH_3$ | S | $N-CH_3$ |
| 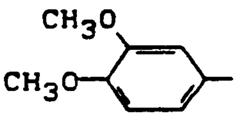 | H | $CH_3$ | $OCH_3$ | S | $N-CH_3$ |
| 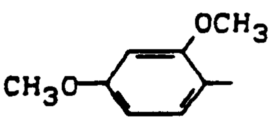 | H | $CH_3$ | $OCH_3$ | S | $N-CH_3$ |
| 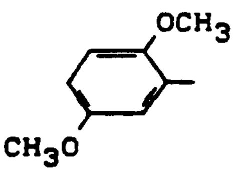 | H | $CH_3$ | $OCH_3$ | S | $N-CH_3$ |
| 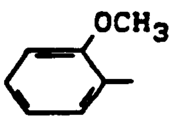 | H | $CH_3$ | $OCH_3$ | S | $N-CH_3$ |

| $R^1$ | $R^2$ | $R^3$ | $R^4$ | X | Y |
|---|---|---|---|---|---|
| 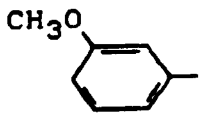 | H | $CH_3$ | $OCH_3$ | S | $N-CH_3$ |
| 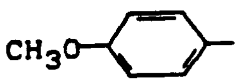 | H | $CH_3$ | $OCH_3$ | S | $N-CH_3$ |
| 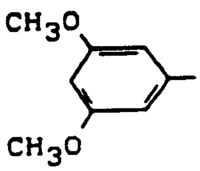 | H | $CH_3$ | $OCH_3$ | S | $N-CH_3$ |
| 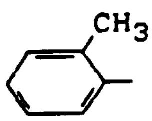 | H | $CH_3$ | $OCH_3$ | S | $N-CH_3$ |
| 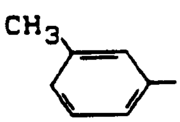 | H | $CH_3$ | $OCH_3$ | S | $N-CH_3$ |
| 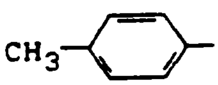 | H | $CH_3$ | $OCH_3$ | S | $N-CH_3$ |
| 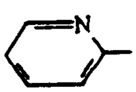 | H | $CH_3$ | $OCH_3$ | S | $N-CH_3$ |
| 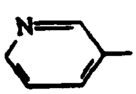 | H | $CH_3$ | $OCH_3$ | S | $N-CH_3$ |
| 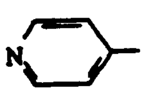 | H | $CH_3$ | $OCH_3$ | S | $N-CH_3$ |
| 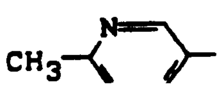 | H | $CH_3$ | $OCH_3$ | S | $N-CH_3$ |

| $R^1$ | $R^2$ | $R^3$ | $R^4$ | X | Y |
|---|---|---|---|---|---|
| 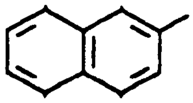 | H | $CH_3$ | $OCH_3$ | S | N-$CH_3$ |
| 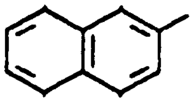 | H | $CH_3$ | $OCH_3$ | O | N-$CH_3$ |
| 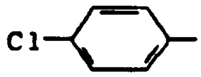 | H | $CH_3$ | $OCH_3$ | S | 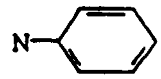 |
| 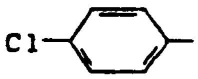 | $CH_3$ | $CH_3$ | $OCH_3$ | S | N-$CH_3$ |
| 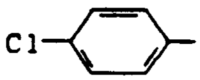 | H | $CH_3$ | $OCH_3$ | O | 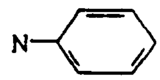 |
| 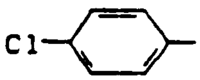 | $CH_3$ | $CH_3$ | $OCH_3$ | O | N-$CH_3$ |
| 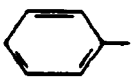 | H | $CH_3$ | $OCH_3$ | O | N-$CH_3$ |
| 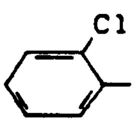 | H | $CH_3$ | $OCH_3$ | O | N-$CH_3$ |
| 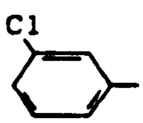 | H | $CH_3$ | $OCH_3$ | O | N-$CH_3$ |
| 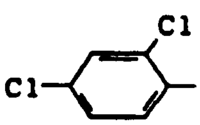 | H | $CH_3$ | $OCH_3$ | O | N-$CH_3$ |

| $R^1$ | $R^2$ | $R^3$ | $R^4$ | X | Y |
|---|---|---|---|---|---|
| 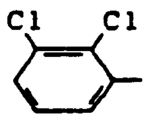 | H | $CH_3$ | $OCH_3$ | O | $N-CH_3$ |
| 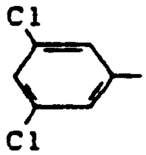 | H | $CH_3$ | $OCH_3$ | O | $N-CH_3$ |
| 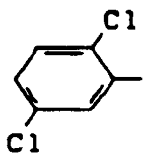 | H | $CH_3$ | $OCH_3$ | O | $N-CH_3$ |
| 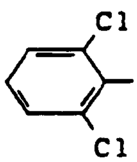 | H | $CH_3$ | $OCH_3$ | O | $N-CH_3$ |
| 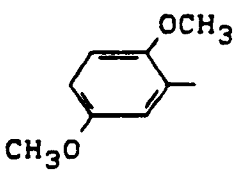 | H | $CH_3$ | $OCH_3$ | O | $N-CH_3$ |
| 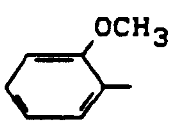 | H | $CH_3$ | $OCH_3$ | O | $N-CH_3$ |
| 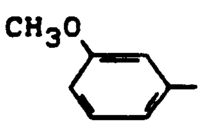 | H | $CH_3$ | $OCH_3$ | O | $N-CH_3$ |
| 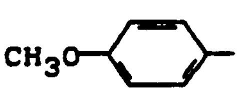 | H | $CH_3$ | $OCH_3$ | O | $N-CH_3$ |

| $R^1$ | $R^2$ | $R^3$ | $R^4$ | X | Y |
|---|---|---|---|---|---|
| 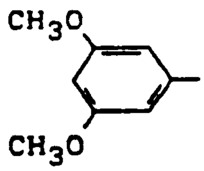 | H | $CH_3$ | $OCH_3$ | O | $N-CH_3$ |
| 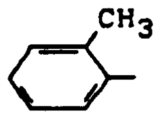 | H | $CH_3$ | $OCH_3$ | O | $N-CH_3$ |
| 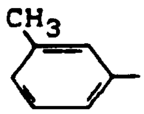 | H | $CH_3$ | $OCH_3$ | O | $N-CH_3$ |
| 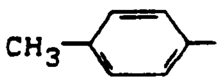 | H | $CH_3$ | $OCH_3$ | O | $N-CH_3$ |
| 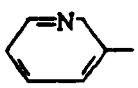 | H | $CH_3$ | $OCH_3$ | O | $N-CH_3$ |
| 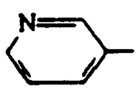 | H | $CH_3$ | $OCH_3$ | O | $N-CH_3$ |
| 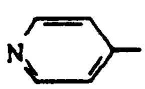 | H | $CH_3$ | $OCH_3$ | O | $N-CH_3$ |
| 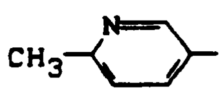 | H | $CH_3$ | $OCH_3$ | O | $N-CH_3$ |
| 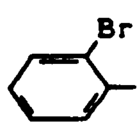 | H | $CH_3$ | $OCH_3$ | O | $N-CH_3$ |

| $R^1$ | $R^2$ | $R^3$ | $R^4$ | X | Y |
|---|---|---|---|---|---|
| 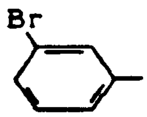 | H | $CH_3$ | $OCH_3$ | O | N-$CH_3$ |
| 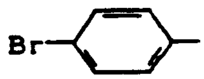 | H | $CH_3$ | $OCH_3$ | O | N-$CH_3$ |
| 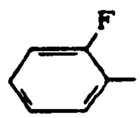 | H | $CH_3$ | $OCH_3$ | O | N-$CH_3$ |
| 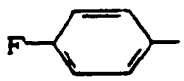 | H | $CH_3$ | $OCH_3$ | O | N-$CH_3$ |
| 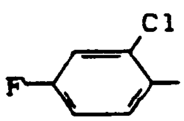 | H | $CH_3$ | $OCH_3$ | O | N-$CH_3$ |
| 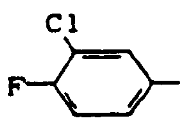 | H | $CH_3$ | $OCH_3$ | O | N-$CH_3$ |
| 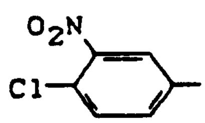 | H | $CH_3$ | $OCH_3$ | O | N-$CH_3$ |
| 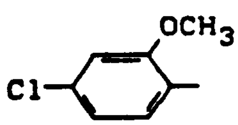 | H | $CH_3$ | $OCH_3$ | O | N-$CH_3$ |
| 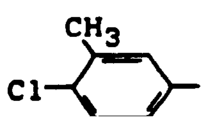 | H | $CH_3$ | $OCH_3$ | O | N-$CH_3$ |

| $R^1$ | $R^2$ | $R^3$ | $R^4$ | X | Y |
|---|---|---|---|---|---|
| 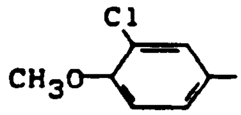 | H | $CH_3$ | $OCH_3$ | O | $N-CH_3$ |
| 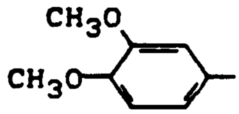 | H | $CH_3$ | $OCH_3$ | O | $N-CH_3$ |
| 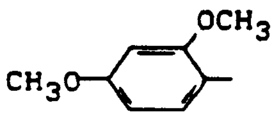 | H | $CH_3$ | $OCH_3$ | O | $N-CH_3$ |
| 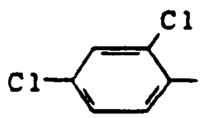 | H | $CH_3$ | $SCH_3$ | S | $N-CH_3$ |
| 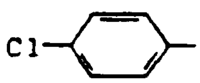 | H | $CH_3$ | $SCH_3$ | S | $N-CH_3$ |
| 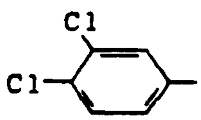 | H | $CH_3$ | $OCH_3$ | S | $N-CH_3$ |
| 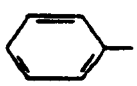 | H | $CH_3$ | $OCH_3$ | S | $N-CH_3$ |
| 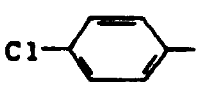 | H | $CH_3$ | $-N{<}^{CH_3}_{CH_3}$ | S | $N-CH_3$ |
| 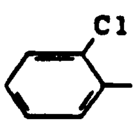 | H | $CH_3$ | $-N{<}^{CH_3}_{CH_3}$ | O | $N-CH_3$ |

| $R^1$ | $R^2$ | $R^3$ | $R^4$ | X | Y |
|---|---|---|---|---|---|
| 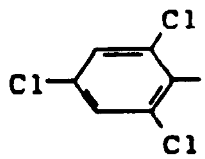 | H | $CH_3$ | $OCH_3$ | S | S |
| 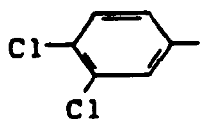 | H | $CH_3$ | $OCH_3$ | O | S |
| 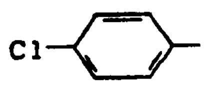 | H | $C_2H_5$ | $OCH_3$ | S | $N-CH_3$ |
| 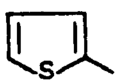 | H | $CH_3$ | $OCH_3$ | S | $N-CH_3$ |
| 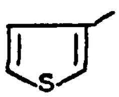 | H | $CH_3$ | $OCH_3$ | S | $N-CH_3$ |
| 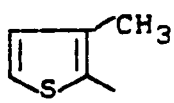 | H | $CH_3$ | $OCH_3$ | S | $N-CH_3$ |
| 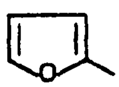 | H | $CH_3$ | $OCH_3$ | S | $N-CH_3$ |
| 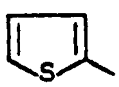 | H | $CH_3$ | $OCH_3$ | O | $N-CH_3$ |
| 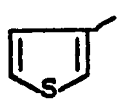 | H | $CH_3$ | $OCH_3$ | O | $N-CH_3$ |
| 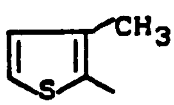 | H | $CH_3$ | $OCH_3$ | O | $N-CH_3$ |
| 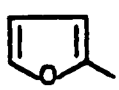 | H | $CH_3$ | $OCH_3$ | O | $N-CH_3$ |

Verwendet man beispielsweise 2-{N-[4-(3,4-Dichlorphenyl)-thiazol-2-yl]-N-methylamino}-3-hydroxy-acrylsäuremethylester und Dimethylsulfat als Ausgangsstoffe, so läßt sich der Reaktionsablauf des erfindungsgemäßen Verfahrens (a) durch das folgende Formelschema darstellen:

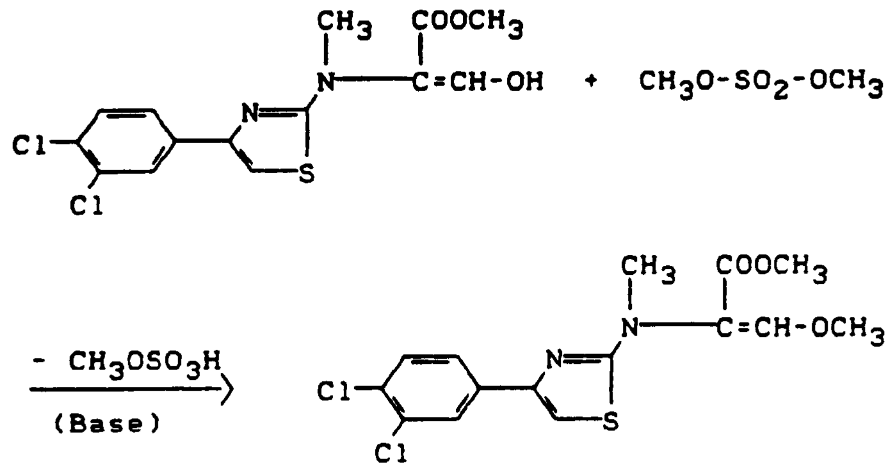


Verwendet man beispielsweise 2-[4-(4-Chlorphenyl)-thiazol-2-yl-oxy]-essigsäuremethylester und Dimethylformamiddimethylacetal als Ausgangsstoffe, so läßt sich der Reaktionsablauf des erfindungsgemäßen Verfahrens (b) durch das folgende Formelschema darstellen:

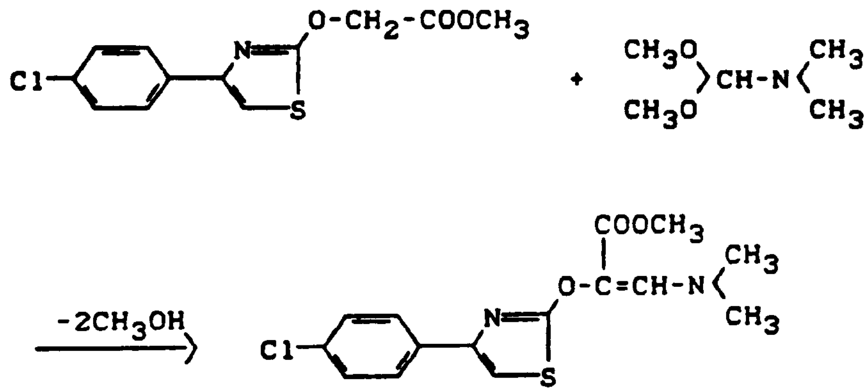


Verwendet man beispielsweise 2-[4-(2,4-Dichlorphenyl)-thiazol-2-yl-thio]-2-oxo-essigsäuremethylester und (Methylthio)-(trimethylsilyl)-methylenlithium als Ausgangsstoffe, so läßt sich der Reaktionsablauf des erfindungsgemäßen Verfahrens (c) durch das folgende Formelschema darstellen:

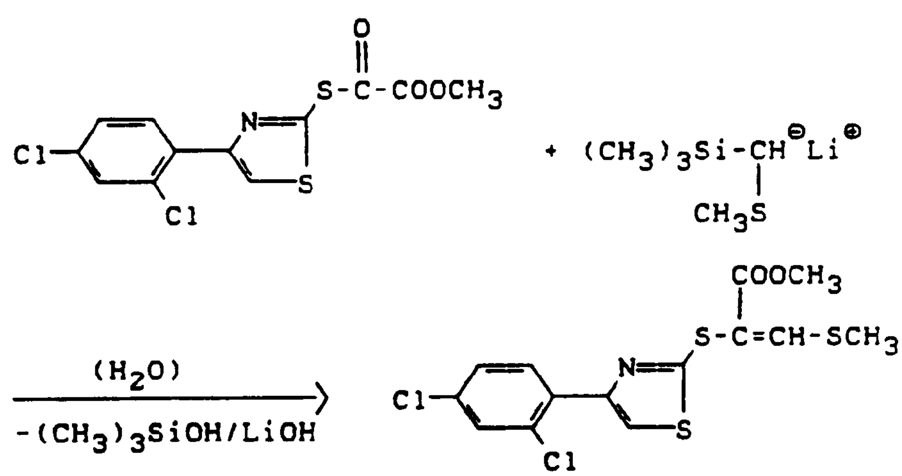


Verwendet man beispielsweise 2-[4-(4-Chlorphenyl)-oxazol-2-yl-oxy]-3-methylsulfonyloxy-acrylsäuremethylester und Methylmercaptan als Ausgangsstoffe, so läßt sich der Reaktionsablauf des erfindungsgemäßen

Verfahrens (d) durch das folgende Formelschema darstellen:

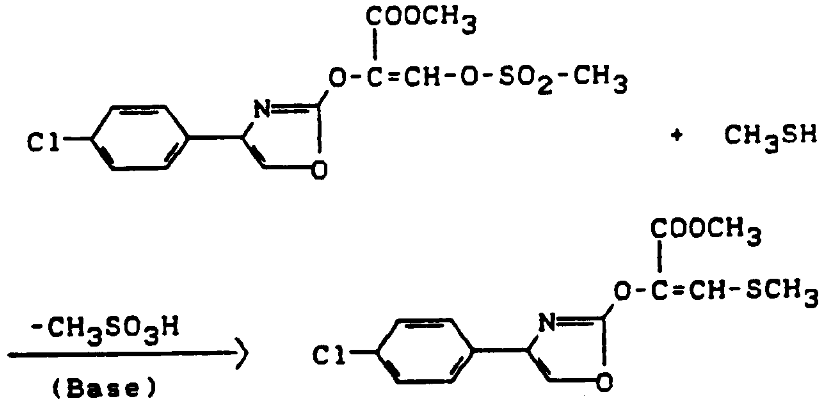


Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen $R^1$, $R^2$, $R^3$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Natrium- oder Kaliumkation.

Die Hydroxyacrylsäureester der Formel (II) sind teilweise bekannt (vgl. EP 61 425).

Noch nicht bekannt und ebenfalls Gegenstand der Erfindung sind Verbindungen der Formel (IIa),

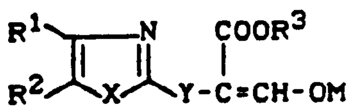 (IIa)

in welcher

$R^1$, $R^2$, $R^3$, X, Y und M die oben angegebene Bedeutung haben,

ausgenommen die Verbindung 2-[4,5-Bis(4-methoxyphenyl)-thiazol-2-yl-thio]-3-hydroxyacrylsäureethylester.

Man erhält sie, wenn man substituierte Essigsäureester der Formel (IV),

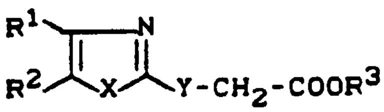 (IV)

in welcher

$R^1$, $R^2$, $R^3$, X und Y die oben angegebene Bedeutung haben, ausgenommen die Verbindung 2-[4,5-Bis(4-methoxyphenyl)-thiazol-2-yl-thio]-essigsäureethylester,

mit Ameisensäureestern der Formel (X),

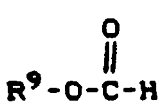 (X)

in welcher

$R^9$ für Alkyl, insbesondere für Methyl oder Ethyl steht,

gegebenenfalls in Gegenwart eines Verdünnungsmittels wie beispielsweise Dimethylformamid und gegebenenfalls in Gegenwart eines basischen Reaktionshilfsmittels wie beispielsweise Natriumhydrid bei Temperaturen zwischen - 20 °C und + 50 °C umsetzt.

Ameisensäureester der Formel (X) sind allgemein bekannte Verbindungen der organischen Chemie.

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester sind durch die Formel (IV) allgemein definiert. In dieser Formel (IV) stehen $R^1$, $R^2$, $R^3$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Essigsäureester der Formel (IV) sind bekannt oder erhältlich in Analogie zu bekannten Verfahren (vgl. z.B. J. Ind. Chem. Soc. 46, 441-443 [1963]; FR 21 52 345; FR 21 46 161; Chem. Ber. 92, 1928 [1959]; Arzneimittel-Forsch. 36, 1391-1393 [1986]. GB 15 52 126; DE-OS 21 29 012 sowie die Herstellungsbeispiele).

Die zur Durchführung des erfindungsgemäßen Verfahrens (b) weiterhin als Ausgangsstoffe benötigten Formamide und deren Derivate sind durch die Formeln (Va) und (Vb) allgemein definiert. In diesen Formeln (Va) und (Vb) steht $R^{4-1}$ vorzugsweise für Dialkylamino mit jeweils 1 bis 6, insbesondere mit 1 bis 4 Kohlenstoffatomen in den einzelnen geradkettigen oder verzweigten Alkylteilen. $R^{4-1}$ steht ganz besonders bevorzugt für Dimethylamino oder Diethylamino.

$R^7$ und $R^8$ stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest, mit jeweils 1 bis 6 insbesondere mit 1 bis 4 Kohlenstoffatomen in den einzelnen geradkettigen oder verzweigten Alkylteilen.

Die Formamide der Formel (V) und deren Derivate der Formel (Vb) sind allgemein bekannte Verbindungen der organischen Chemie.

Die zur Durchführung des erfindungsgemäßen Verfahrens (c) als Ausgangsstoffe benötigten Oxalsäurederivate sind durch die Formel (VI) allgemein definiert. In dieser Formel (VI) stehen $R^1$, $R^2$, $R^3$ und X vorzugsweise für diejenigen Reste, die bereits im Zusammenhang mit der Beschreibung der erfindungsgemäßen Stoffe der Formel (I) als bevorzugt für diese Substituenten genannt wurden.

$Y^1$ steht vorzugsweise für Schwefel oder für einen Rest

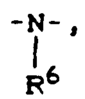

wobei $R^6$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die Oxalsäurederivate der Formel (VI) sind größtenteils bekannt (vgl. z.B. Arzneimittel-Forsch. 36, 1391-1393 [1986]; J. med . chem. 26, 1158-1163 [1983]; DE-OS 28 28 091; EP 6368) oder erhältlich in Analogie zu bekannten Verfahren (vgl. z.B. Synthetic Communications 11, 943 [1981] oder Organic Reactions 26, 1 [1979]), beispielsweise wenn man Oxalester der Formel (XI),

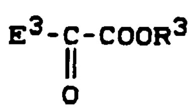

(XI)

in welcher

$E^3$ für Alkoxy oder Halogen insbesondere für Methoxy, Ethoxy oder Chlor steht und

$R^3$ die oben angegebene Bedeutung hat,

mit Heterocyclen der Formel (XII),

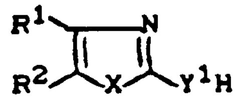

(XII)

in welcher

$R^1$, $R^2$, X und $Y^1$ die oben angegebene Bedeutung haben,

gegebenenfalls in Gegenwart eines Verdünnungsmittels, wie beispielsweise Dichlormethan oder Tetrahydrofuran und gegebenenfalls in Gegenwart einer Base, wie beispielsweise n-Butyllithium, Natriumhydrid, Kalium-t-butylat, Triethylamin oder Pyridin bei Temperaturen zwischen - 80 °C und + 80 °C umsetzt.

Oxalester der Formel (XI) sind allgemein bekannte Verbindungen der organischen Chemie.

Heterocyclen der Formel (XII) sind ebenfalls allgemein bekannt oder erhältlich in Analogie zu allgemein bekannten Verfahren (vgl. z.B. Organic Reactions 6, 367 ff. sowie die Herstellungsbeispiele).

Die zur Durchführung des erfindungsgemäßen Verfahrens (c) weiterhin als Ausgangsstoffe benötigten metallorganischen Verbindungen sind durch die Formel (VII) allgemein definiert. In dieser Formel (VII) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

Die metallorganischen Verbindungen der Formel (VII) sind bekannt (vgl. z.B. J. org. Chem. 33, 780 [1968]; J.org. Chem. 37, 939 [1972]).

Die zur Durchführung des erfindungsgemäßen Verfahrens (d) als Ausgangsstoffe benötigten substituierten Acrylsäureester sind durch die Formel (VIII) allgemein definiert. In dieser Formel (VIII) stehen $R^1$, $R^2$, $R^3$ und X vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht vorzugsweise für einen geeigneten Acyloxy- oder Sulfonyloxyrest, insbesondere für einen Acetoxy-, einen Methansulfonyloxy- oder einen p-Toluolsulfonyloxyrest.

Die substituierten Acrylsäureester der Formel (VIII) sind noch nicht bekannt.

Man erhält sie, wenn man Hydroxyacrylsäureester der Formel (IIb),

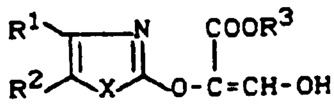

(IIb)

in welcher

$R^1$, $R^2$, $R^3$ und X die oben angegebene Bedeutung haben,

mit Säurechloriden der Formel (XIII),

$$R^{10}\text{-Cl} \qquad \text{(XIII)}$$

in welcher

$R^{10}$ für einen Acyl- oder Sulfonylrest, insbesondere für einen Acetyl-, einen Methansulfonyl- oder ein p-Toluolsulfonylrest steht,

gegebenenfalls in Gegenwart eines Verdünnungsmittels wie beispielsweise Dichlormethan und gegebenenfalls in Gegenwart eines Säurebindemittels wie beispielsweise Triethylamin oder Pyridin bei Temperaturen zwischen - 20 °C und + 120 °C umsetzt.

Säurechloride der Formel (XIII) sind allgemein bekannte Verbindungen der organischen Chemie.

Die zur Durchführung des erfindungsgemäßen Verfahrens (d) weiterhin als Ausgangsstoffe benötigten Thiole sind durch die Formel (IX) allgemein definiert. In dieser Formel (IX) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thiole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen - 30 °C und + 120 °C, vorzugsweise bei Temperaturen zwischen - 20 °C und + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyloder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C und + 200 °C, vorzugsweise bei Temperaturen zwischen 0 °C und 150 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid der Formel (Va) oder eines entsprechenden Derivates der Formel (Vb) ein. Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol. I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan oder Ether, wie Diethylether, Dioxan, Tetrahydrofuran oder Ethylenglykoldimethyloder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen - 100 °C und + 100 °C, vorzugsweise bei Temperaturen zwischen - 80 °C und + 50 °C.

Zur Durchführung des erfindungsgemäßen Verfahrens (c) setzt man pro Mol an Oxalsäurederivat der Formel (VI) im allgemeinen 1.0 bis 1.5 Mol, vorzugsweise 1.0 bis 1.2 Mol an metallorganischer Verbindung der Formel (VII) ein.
Die Reaktionsdurchführung, Aufarbeitung und Isolierung der Reaktionsprodukte erfolgt nach bekannten Verfahren (vgl. z.B. J. org. Chem. 33, 780 [1968]; J. org. Chem. 37; 939 [1972]).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, oder tertiäre Amine, wie Triethylamin, N,N-Dimethylanilin, Pyridin, N,N-Dimethylaminopyridin, Diazabicyclooctan (DABCO), Diazabicyclo0nonen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen - 20 °C und 180 °C, vorzugsweise bei Temperaturen zwischen 0 °C und 150 °C.

Das erfindungsgemäße Verfahren kann in Abhängigkeit vom Siedepunkt der verwendeten Reaktionspartner, beispielsweise beim Einsatz von niedrigsiedenden Thiolen der Formel (IX) gegebenenfalls auch unter Druck durchgeführt werden.
Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (d) setzt man pro Mol an substituiertem Acrylsäureester der Formel (VIII) im allgemeinen 1.0 bis 20.0 Mol, vorzugsweise 1.0 bis 5.0 Mol an Thiol der Formel (IX) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) oder zur Bekämpfung von Reiskrankheiten, wie beispielsweise gegen den Erreger der Reisfleckenkrankheit

(Pyricularia oryzae) oder zur Bekämpfung von Krankheiten im Obst- und Gemüseanbau, wie beispielsweise gegen den Erreger des Apfelschorfes (Venturia inaequalis) oder gegen den Erreger der Tomatenbraunfäule (Phytophthora infestans) sowie gegen Cercospora-Arten an Bohnen einsetzen.

Darüberhinaus zeigen die erfindungsgemäßen Wirkstoffe eine gute in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

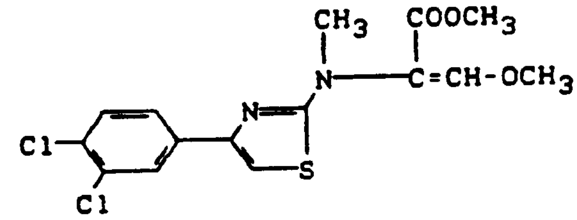

(Verfahren a)

Zu 13 g (0.035 Mol) 2-{N-[4-(3,4-Dichlorphenyl)-thiazol-2-yl]-N-methylamino}-3-hydroxy-acrylsäuremethylester in 60 ml trockenem Dimethylformamid gibt man zunächst 19,3 g (0.14 Mol) gemahlenes Kaliumcarbonat und anschließend tropfenweise unter Rühren im Verlauf von 10 Minuten 4,63 g (0.037 Mol) Dimethylsulfat in 10 ml trockenem Dimethylformamid und rührt dann 16 Stunden bei Raumtemperatur. Zur Aufarbeitung gibt man die Reaktionsmischung in 70 ml gesättigte wässrige Natriumhydrogencarbonatlösung, extrahiert dreimal mit jeweils 100 ml Diethylether, wäscht die vereinigten Etherphasen mit 80 ml gesättigter wässriger Natriumchloridlösung, trocknet über Natriumsulfat, engt im Vakuum ein und reinigt den Rückstand durch Chromatographie an Kieselgel (Laufmittel: Toluol/n-Propanol 10:1).

Man erhält 9,6 g (74 % der Theorie) an 2-{N-[4-(3,4-Dichlorphenyl)-thiazol-2-yl]-N-methylamino}-3-methoxyacrylsäuremethylester vom Schmelzpunkt 96 °C - 98 °C.

Herstellung der Ausgangsverbindung

Beispiel II-1

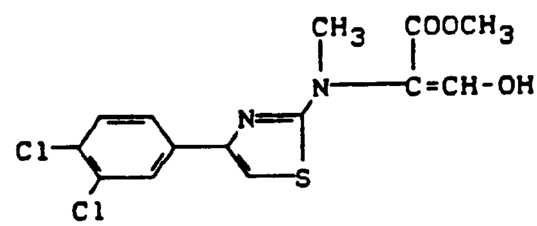

Zu einer Suspension von 2,1 g (0.07 Mol) Natriumhydrid (80prozentig in Paraffin) in 40 ml trockenem Dimethylformamid gibt man bei 5 °C bis 10 °C tropfenweise unter Rühren und Eiskühlung 11,6 g (0.035 Mol) 2-{N-[4-(3,4-Dichlorphenyl)-thiazol-2-yl]-N-methylamino}-essigsäuremethylester in 69,7 g (1.16 Mol) trockenem Ameisensäuremethylester und rührt nach beendeter Zugabe 4 Stunden bei Raumtemperatur. Zur Aufarbeitung gibt man die Reaktionsmischung unter Rühren und Eiskühlung in 50 ml gesättigte wässrige Natriumcarbonatlösung, verdünnt mit 50 ml Wasser, wäscht dreimal mit jeweils 75 ml Diethylether, säuert die wässrige Phase mit 5N-Salzsäure auf pH 5 an und extrahiert dreimal mit jeweils 100 ml Diethylether. Die vereinigten Etherphasen werden über Natriumsulfat getrocknet und im Vakuum eingeengt.

Man erhält 13 g (100 % der Theorie) an 2-{N-[4-(3,4-Dichlorphenyl)-thiazol-2-yl]-N-methylamino}-3-hydroxyacrylsäuremethylester als Öl.

$^1$H-NMR (DMSO/Tetramethylsilan): $\delta$ = 12 ppm (OH).

Beispiel IV-1

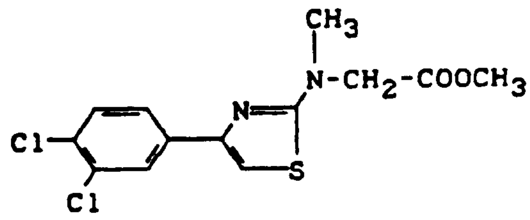

Zu einer Suspension von 27,6 g (0.195 Mol) gemahlenem Kaliumcarbonat und 50,5 g (0.195 Mol) 4-(3,4-Dichlorphenyl)-2-methylaminothiazol in 350 ml trockenem Acetonitril gibt man bei 80 °C tropfenweise unter Rühren innerhalb von 20 Minuten 33,7 g (0.22 Mol) Bromessigsäuremethylester und erhitzt anschließend für 26 Stunden auf Rückflußtemperatur. Zur Aufarbeitung wird die abgekühlte Reaktionsmischung filtriert, das Filtrat im Vakuum eingeengt, der Rückstand in Dichlormethan aufgenommen, mehrfach mit Wasser gewaschen, über Natriumsulfat getrocknet, im Vakuum eingeengt und der Rückstand an Kieselgel chromatographiert (Laufmittel: Toluol/Propanol 10:1).

Man erhält 14,6 g (22 % der Theorie) an 2-{N-[4-(3,4-Dichlorphenyl)-thiazol-2-yl]-N-methyl-amino}-essigsäuremethylester vom Schmelzpunkt 88 °C - 89 °C.

Beispiel XII-1

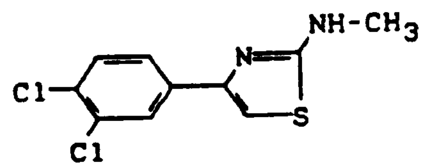

Zu einer 60 °C - 80 °C heißen Lösung von 27,1 g (0.3 Mol) N-Methylthioharnstoff in 300 ml Ethanol gibt man innerhalb von 25 Minuten bei Raumtemperatur tropfenweise unter Rühren 67,05 g (0.3 Mol) 3,4-Dichlorphenacylchlorid in 300 ml Ethanol, erhitzt anschließend 1 Stunde auf Rückflußtemperatur, läßt dann langsam auf Raumtemperatur abkühlen, filtriert den kristallinen Niederschlag ab und kristallisiert aus Ethanol um.

Man erhält 70,9 g (91 % der Theorie) an 4-(3,4-Dichlorphenyl)-2-methylaminothiazol vom Schmelzpunkt 220 °C - 221 °C.

In entsprechender Weise und gemäß den allgemeinen Angaben zur Herstellung erhält man die folgenden substituierten Acrylsäureester der allgemeinen Formel (I):

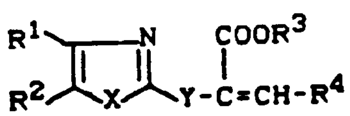

(I)

| Bsp. Nr. | $R^1$ | $R^2$ | $R^3$ | $R^4$ | X | Y | physikalische Eigenschaften |
|---|---|---|---|---|---|---|---|
| 2 | 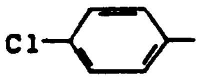 | H | $CH_3$ | $OCH_3$ | S | N-$CH_3$ | Fp.104-105° C |
| 3 | 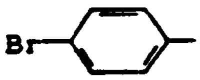 | H | $CH_3$ | $OCH_3$ | S | N-$CH_3$ | Fp.108-109° C |
| 4 | 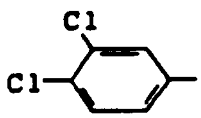 | H | $CH_3$ | $OCH_3$ | S | 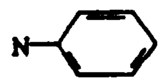 | Fp.154 °C |
| 5 | 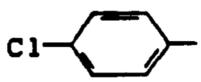 | H | $CH_3$ | $OCH_3$ | O | N-$CH_3$ | Fp.87-88° C |
| 6 | 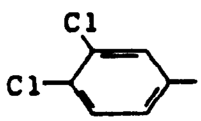 | H | $CH_3$ | $OCH_3$ | O | N-$CH_3$ | $^1$H-NMR*): 3,28; 3,73; 3,92; 7,3-7,8 |
| 7 | 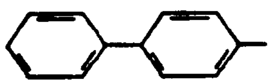 | H | $CH_3$ | $OCH_3$ | S | N-$CH_3$ | $n_D^{22.5}$ 1.5941 |
| 8 | 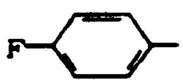 | H | $CH_3$ | $OCH_3$ | S | N-$CH_3$ | Fp.66-67° C |
| 9 | 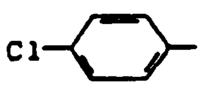 | H | $CH_3$ | $OCH_3$ | S | 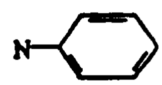 | Fp.68 °C |
| 10 | 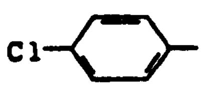 | $CH_3$ | $CH_3$ | $OCH_3$ | S | N-$CH_3$ | $n_D^{20}$ 1.5915 |
| 11 | 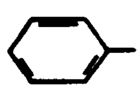 | H | $CH_3$ | $OCH_3$ | S | N-$CH_3$ | Fp.81-82 °C |

| Bsp. Nr. | $R^1$ | $R^2$ | $R^3$ | $R^4$ | X | Y | physikalische Eigenschaften |
|---|---|---|---|---|---|---|---|
| 12 | 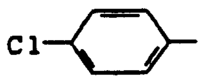 | H | $CH_3$ | $OCH_3$ | S | S | Fp. 89-90° C |
| 13 | 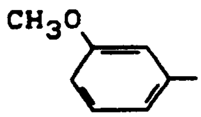 | H | $CH_3$ | $OCH_3$ | S | N-$CH_3$ | $n_D^{20}$ 1.6065 |
| 14 | 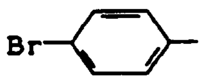 | H | $CH_3$ | $OCH_3$ | O | N-$CH_3$ | Fp.102° C |
| 15 | 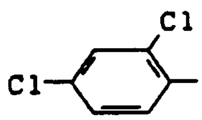 | H | $CH_3$ | $OCH_3$ | S | N-$CH_3$ | $n_D^{20}$ 1.6111 |
| 16 | 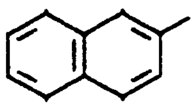 | H | $CH_3$ | $OCH_3$ | S | N-$CH_3$ | $n_D^{20}$ 1.6258 |
| 17 | 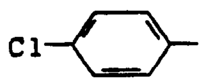 | H | $CH_3$ | $OCH_3$ | S | N-$CH_2$-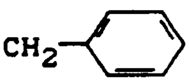 | Fp.60-61° C |
| 18 | 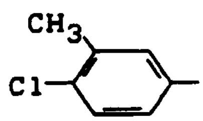 | H | $CH_3$ | $OCH_3$ | S | N-$CH_3$ | Fp.123-124° C |
| 19 | 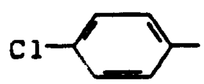 | H | $CH_3$ | $OCH_3$ | S | N-$C_2H_5$ | $n_D^{20}$ 1.6043 |
| 20 | 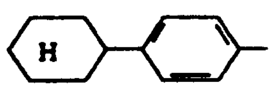 | H | $CH_3$ | $OCH_3$ | S | N-$CH_3$ | Fp.138-139° C |
| 21 | 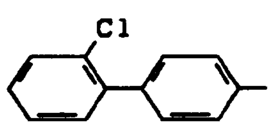 | H | $CH_3$ | $OCH_3$ | S | N-$CH_3$ | $n_D^{20}$ 1.6178 |

| Bsp. Nr. | $R^1$ | $R^2$ | $R^3$ | $R^4$ | X | Y | physikalische Eigenschaften |
|---|---|---|---|---|---|---|---|
| 22 | 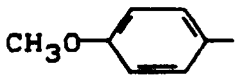 | H | $CH_3$ | $OCH_3$ | S | $N-CH_3$ | Fp. 100-101°C |
| 23 | 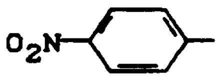 | H | $CH_3$ | $OCH_3$ | S | $N-CH_3$ | Fp. 118-119° C |
| 24 | 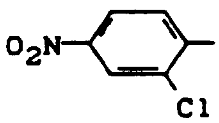 | H | $CH_3$ | $OCH_3$ | S | $N-CH_3$ | Fp. 128-129° C |
| 25 | 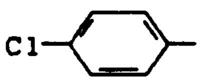 | H | $CH_3$ | $OCH_3$ | S | O | Fp. 114-115° C |
| 26 | 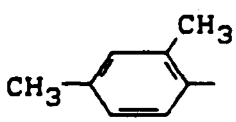 | H | $CH_3$ | $OCH_3$ | S | $N-CH_3$ | $n_D^{20}$ 1.5943 |
| 27 | 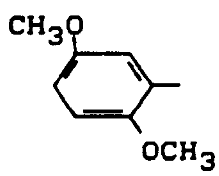 | H | $CH_3$ | $OCH_3$ | S | $N-CH_3$ | $n_D^{20}$ 1.6073 |
| 28 | 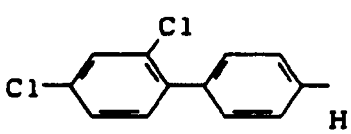 | H | $CH_3$ | $OCH_3$ | S | $N-CH_3$ | Fp. 126-127° C |
| 29 | 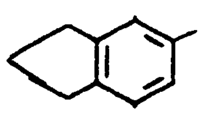 | H | $CH_3$ | $OCH_3$ | S | $N-CH_3$ | Fp. 95-96° C |
| 30 | 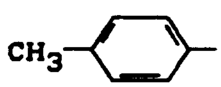 | H | $CH_3$ | $OCH_3$ | S | $N-CH_3$ | Fp. 115-116° C |
| 31 | 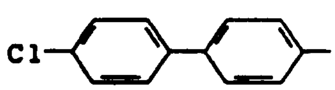 | H | $CH_3$ | $OCH_3$ | S | $N-CH_3$ | Fp. 182-183° C |

| Bsp. Nr. | $R^1$ | $R^2$ | $R^3$ | $R^4$ | X | Y | physikalische Eigenschaften |
|---|---|---|---|---|---|---|---|
| 32 | 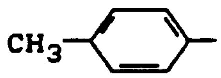 | 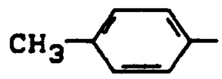 | $CH_3$ | $OCH_3$ | S | N-$CH_3$ | $n_D^{20}$ 1.6059 |
| 33 | 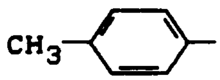 | $CH_3$ | $CH_3$ | $OCH_3$ | S | N-$CH_3$ | Fp.67-69° C |
| 34 | 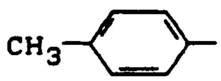 | H | $CH_3$ | $OCH_3$ | S | S | Fp.89-90° C |
| 35 | 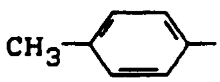 | $C_2H_5$ | $CH_3$ | $OCH_3$ | S | N-$CH_3$ | $n_D^{20}$ 1.6109 |
| 36 | 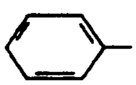 | $CH_3-(CH_2)_2-$ | $CH_3$ | $OCH_3$ | S | N-$CH_3$ | $n_D^{20}$ 1.5755 |
| 37 | 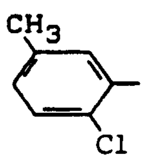 | H | $CH_3$ | $OCH_3$ | S | S | $n_D^{20}$ 1.6171 |
| 38 | 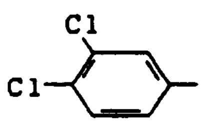 | H | $CH_3$ | $OCH_3$ | S | S | Fp.110-111° C |
| 39 | 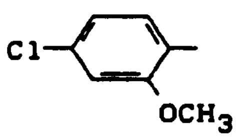 | H | $CH_3$ | $OCH_3$ | S | N-$CH_3$ | Fp.104-105° C |
| 40 | 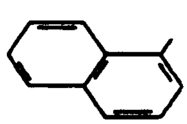 | H | $CH_3$ | $OCH_3$ | S | N-$CH_3$ | $n_D^{20}$ 1.6073 |

| Bsp. Nr. | $R^1$ | $R^2$ | $R^3$ | $R^4$ | X | Y | physikalische Eigenschaften |
|---|---|---|---|---|---|---|---|
| 41 | 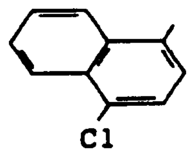 | H | $CH_3$ | $OCH_3$ | S | $N-CH_3$ | $n_D^{20}$ 1.5982 |
| 42 | 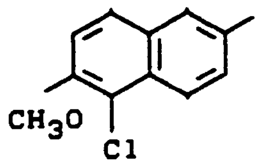 | H | $CH_3$ | $OCH_3$ | S | $N-CH_3$ | Fp.154-155°C |
| 43 | 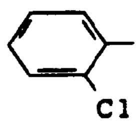 | H | $CH_3$ | $OCH_3$ | O | $N-CH_3$ | $^1$H-NMR*): 3.3(s); 3.73(s); 3.9(s); |
| 44 | 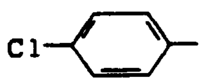 | $C_2H_5$ | $CH_3$ | $OCH_3$ | O | $N-CH_3$ | $^1$H-NMR*): 1.2(t); 2.71(t); 2.20(c); 3.71(c); |

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

## Anwendungsbeispiele

In den folgenden Anwendungsbeispielen wurden die nachstehend aufgeführten Verbindungen als Vergleichsubstanzen eingesetzt:

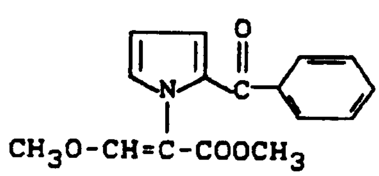

(A)

3-Methoxy-2-(2-benzoylpyrrol-1-yl)-acrylsäure-methylester
(bekannt aus EP 206 523)

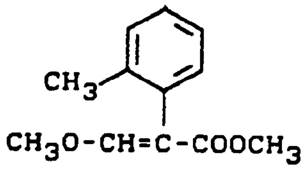

(B)

3-Methoxy-2-(2-methylphenyl)-acrylsäuremethylester
(bekannt aus EP 178 816)

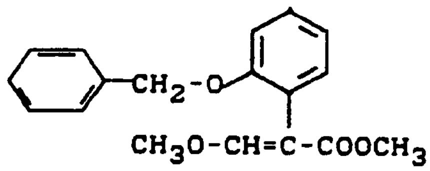

(C)

3-Methoxy-2-(2-benzyloxyphenyl)-acrylsäuremethylester
(bekannt aus DE-OS 35 19 282).

Beispiel A

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß Herstellungsbeispiel 1.

Beispiel B

Phytophthora-Test (Tomate)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigt in diesem Test z.B. die Verbindung gemäß Herstellungsbeispiel 1.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 ° C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß Herstellungsbeispiel 1.

Beispiel D

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man jungen Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 1.

## Patentansprüche

### Patentansprüche für folgende Vertragsstaaten : BE, CH, DE, FR, GB, IT, LI, NL

1. Substituierte Acrylsäureester der allgemeinen Formel (I),

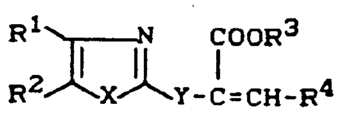

(I)

in welcher

$R^1$ und $R^2$ unabhängig voneinander jeweils für Wasserstoff, für geradkettiges oder verzweigtes Alkyl mit 1 bis 8 Kohlenstoffatomen, für geradkettiges oder verzweigtes Alkenyl mit 2 bis 8 Kohlenstoffatomen, für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder Aryl mit 6 bis 10 Kohlenstoffatomen im jeweiligen Arylteil stehen, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8

Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich öder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verchiedenen Halogenatomen substituiertes Heteroarylalkyl oder Heteroaryl mit jeweils 2 bis 9 Kohlenstoffatomen und 1 bis 4 gleichen oder verschiedenen Heteroatomen im Heteroarylteil und gegebenenfalls 1 bis 4 Kohlenstoffatomen im geradkettigen oder verzweigten Alkylteil;
außerdem für einen gegebenenfalls einfach oder mehrfach, gleich oder verschieden substituierten Heteroarylrest mit 2 bis 9 Kohlenstoffatomen und 1 bis 4 gleichen oder verschiedenen Heteroatomen stehen, wobei als Substituenten die oben genannten Arylsubstituenten infrage kommen,

$R^3$ für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ und $R^2$ genannten infrage kommen,

$R^4$ für Dialkylamino mit jeweils 1 bis 6 Kohlenstoffatomen in den einzelnen Alkylteilen steht oder für einen Rest -Z-$R^5$ steht,

X für Sauerstoff oder Schwefel steht und

Y für Sauerstoff, Schwefel oder für einen Rest

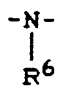

steht,
wobei

$R^5$ für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ und $R^2$ genannten infrage kommen,

$R^6$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil oder Aryl mit jeweils 6 bis 10 Kohlenstoffatomen im jeweiligen Arylteil steht, wobei als Substituenten im Arylteil jeweils die bei $R^1$ und $R^2$ genannten infrage kommen und

Z für Sauerstoff oder Schwefel steht.

2. Substituierte Acrylsäureester gemäß Anspruch 1, wobei in der Formel (I)

$R^1$ und $R^2$ unabhängig voneinander jeweils für Wasserstoff, Methyl, Ethyl, n- oder i-Propyl, n-, i-, s-oder t-Butyl, für Allyl, n- oder i- Butenyl , für jeweils gegebenenfalls im Arylteil oder im Heteroarylteil ein- bis dreifach, gleich oder verschieden substituiertes Benzyl, Phenylethyl, Phenylethenyl, Phenyl, Naphthyl, Pyridyl, Thienyl oder Furyl stehen, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen,

$R^4$ für Dialkylamino mit jeweils 1 bis 4 Kohlenstoffatomen in den einzelnen Alkylteilen steht oder

für einen Rest -Z-$R^5$ steht,

X für Sauerstoff oder Schwefel steht und

Y für Sauerstoff, Schwefel oder für einen Rest

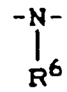

steht,

wobei

$R^5$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen;

$R^6$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ und $R^2$ genannten infrage kommen und

Z für Sauerstoff oder Schwefel steht.

3. Substituierte Acrylsäureester gemäß Anspruch 1, wobei in der Formel

$R^1$ und $R^2$ unabhängig voneinander jeweils für Wasserstoff, Methyl, Ethyl, n- oder i-Propyl oder für jeweils gegebenenfalls ein- bis dreifach, gleich oder verschieden substituiertes Phenyl oder Naphthyl stehen, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n-oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy,

$R^3$ für Methyl, Ethyl oder Benzyl steht,

$R^4$ für Dimethylamino, Diethylamino oder für einen Rest -Z-$R^5$ steht,

X für Sauerstoff oder Schwefel steht und

Y für Sauerstoff, Schwefel oder für einen Rest

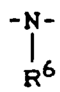

steht,

wobei

$R^5$ für Methyl, Ethyl, n- oder i-Propyl oder Benzyl steht,

$R^6$ für Wasserstoff, Methyl, Ethyl, Acetyl, Propionyl oder für jeweils gegebenenfalls einfach oder zweifach, gleich oder verschieden durch Fluor, Chlor, Brom, Methyl, Ethyl und/ oder Trifluormethyl substituiertes Benzyl oder Phenyl steht und

Z für Sauerstoff oder Schwefel steht.

4. Substituierte Acrylsäurester gemäß Anspruch 1, wobei in der Formel (I)

$R^1$ für Wasserstoff, Methyl, Ethyl, n- oder i- Propyl oder für gegebenenfalls einfach oder zweifach, gleich oder verschieden substituiertes Phenyl steht, wobei als Substituenten infrage kommen:

Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Methoximinoethyl, Cyclopentyl, 1,3-Propandiyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Wasserstoff, Methyl, Ethyl, n- oder i- Propyl steht,

$R^3$ für Methyl oder Ethyl steht,

$R^4$ für Methoxy, Ethox, Methylthio oder Dimethylamino steht,

X für Sauerstoff oder Schwefel steht und

Y für einen N-Methyl-Rest steht.

5. Verfahren zur Herstellung von substituierten Acrylsäureestern der allgemeinen Formel (I),

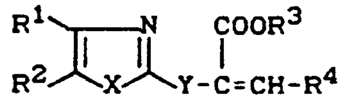 (I)

in welcher $R^1$, $R^2$, $R^3$,$R^4$, X und Y die in Anspruch 1 gegebenen Bedeutungen haben, dadurch gekennzeichnet, daß man

(a) substituierte Acrylsäureester der Formel (Ia),

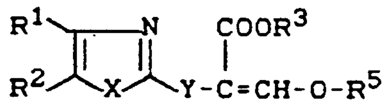 (Ia)

in welcher

$R^1$, $R^2$, $R^3$, $R^5$, X und Y die oben angegebene Bedeutung haben, erhält, wenn man Hydroxyacrylsäureester oder deren Alkalimetallsalze der Formel (II),

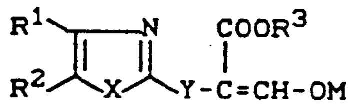 (II)

in welcher

M für Wasserstoff oder für ein Alkalimetallkation steht und

$R^1$, $R^2$, $R^3$, X und Y die oben angegebene Bedeutung haben,

mit Alkylierungsmitteln der Formel (III),

$$R^5\text{-}E^1 \qquad \text{(III)}$$

in welcher

$E^1$ für eine elektronenanziehende Abgangsgruppe steht und

$R^5$ die oben angegebene Bedeutung hat,

gegebenenfalls in Gegenwart eines Verdünnungsmittels und gegebenenfalls in Gegenwart eines Reaktionshilfsmittels umsetzt oder

(b) substituierte Acrylsäureester der Formel (Ib),

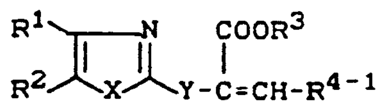 (Ib)

in welcher

$R^{4-1}$ für Dialkylamino steht und

$R^1$, $R^2$, $R^3$, X und Y die oben angegebene Bedeutung haben, erhält, wenn man substituierte Essigsäureester der Formel (IV),

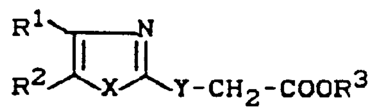 (IV)

in welcher

$R^1$, $R^2$, $R^3$, X und Y die oben angegebene Bedeutung haben,

mit Formamiden der Formel (Va),

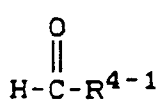 (Va)

in welcher

$R^{4-1}$ die oben angegebene Bedeutung hat,
oder mit deren Derivaten der Formel (Vb),

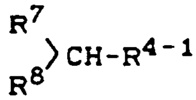

(Vb)

in welcher

$R^7$ und $R^8$ unabhängig voneinander jeweils für Alkoxy oder Dialkylamino stehen und
$R^{4-1}$ die oben angegebene Bedeutung hat,
gegebenenfalls in Gegenwart eines Verdünnungsmittels umsetzt oder

(c) substituierte Acrylsäureester der Formel (Ic),

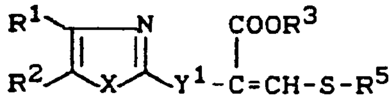

(Ic)

in welcher

$Y^1$ für Schwefel oder für einen Rest

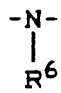

steht,

und

$R^1$, $R^2$, $R^3$, $R^5$, $R^6$ und X die oben angegebene Bedeutung haben, erhält,
wenn man Oxalsäurederivate der Formel (VI),

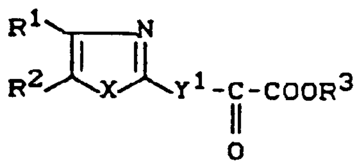

(VI)

in welcher

$R^1$, $R^2$, $R^3$, X und $Y^1$ die oben angegebene Bedeutung haben,
mit metallorganischen Verbindungen der Formel (VII),

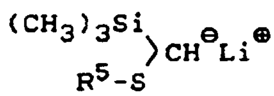

(VII)

in welcher

$R^5$ die oben angegebene Bedeutung hat,
gegebenenfalls in Gegenwart eines Verdünnungsmittels umsetzt oder

(d) substituierte Acrylsäureester der Formel (Id),

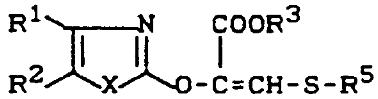

(Id)

in welcher

$R^1$, $R^2$, $R^3$, $R^5$ und X die oben angegebene Bedeutung haben, erhält, wenn man substituierte Acrylsäureester der Formel (VIII),

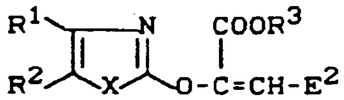

(VIII)

in welcher

$E^2$ für eine elektronenanziehende Abgangsgruppe steht und $R^1$, $R^2$, $R^3$ und X die oben angegebene Bedeutung haben, mit Thiolen der Formel (IX),

$R^5$-SH (IX)

in welcher

$R^5$ die oben angegebene Bedeutung hat, gegebenenfalls in Gegenwart eines Verdünnungsmittels und gegebenenfalls in Gegenwart eines Reaktionshilfsmittels umsetzt.

**6.** Schädlingsbekämpfungsmittel, gekennzeichnet durch einen Gehalt an mindestens einem substituierten Acrylsäureester der Formel (I) nach den Ansprüchen 1 oder 5.

**7.** Verwendung von substituierten Acrylsäureester der Formel (I) nach den Ansprüchen 1 oder 5 zur Bekämpfung von Schädlingen.

**8.** Verfahren zur Bekämpfung von Schädlingen, dadurch gekennzeichnet, daß man substituierte Acrylsäureester der Formel (I) nach den Ansprüchen 1 oder 5 auf Schädlinge und/oder ihren Lebensraum einwirken läßt.

**9.** Verfahren zur Herstellung von Schädlingsbekämpfungsmitteln, dadurch gekennzeichnet, daß man substituierte Acrylsäureester der Formel (I) nach den Ansprüchen 1 oder 5 mit Streckmitteln und/oder oberflächenaktiven Mitteln vermischt.

**10.** Hydroxyacrylsäureester der Formel (IIa),

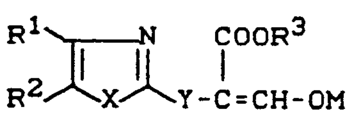

(IIa)

in welcher $R^1$, $R^2$, $R^3$, X, Y die in Anspruch 1 gegebenen Bedeutungen haben, und

M für Wasserstoff oder für ein Alkalimetallkation steht, ausgenommen die Verbindung 2-[4,5-Bis(4-methoxyphenyl)-thiazol-2-yl-thio]-3-hydroxyacrylsäureethylester.

**11.** Verfahren zur Herstellung von Hydroxyacrylsäureestern der Formel (IIa),

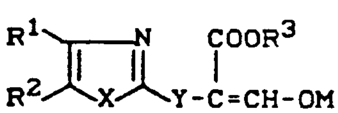

(IIa)

in welcher $R^1$, $R^2$, $R^3$, M, X und Y die in Anspruch 10 gegebenen Bedeutungen haben,

dadurch gekennzeichnet, daß man substituierte Essigsäureester der Formel (IV),

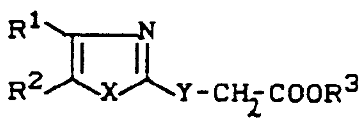

(IV)

in welcher

$R^1$, $R^2$, $R^3$, X und Y die oben angegebene Bedeutung haben,

mit Ameisensäureestern der Formel (X),

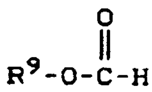

(X)

in welcher

$R^9$ für Alkyl, steht,

gegebenenfalls in Gegenwart eine Verdünnungsmittels und gegebenenfalls in Gegenwart eines basischen Reaktionshilfsmittels bei Temperaturen zwischen -20° C und +50° C umsetzt.

**12.** Substituierte Acrylsäureester der Formel (VIII),

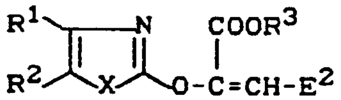

(VIII)

in welcher $R^1$, $R^2$, $R^3$, X die in Anspruch 1 gegebenen Bedeutungen haben, und
$E^2$ für eine elektroneanziehende Abgangsgruppe steht.

**13.** Verfahren zur Herstellung von substituierten Acrylsäureestern der Formel (VIII),

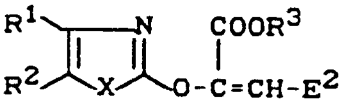

(VIII)

in welcher $R^1$, $R^2$, $R^3$, X und $E^2$die in Anspruch 12 gegebenen Bedeutungen haben,

dadurch gekennzeichnet, daß man Hydroxyacrylsäureester der Formel (IIb),

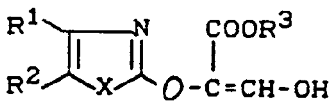

(IIb)

in welcher

$R^1$, $R^2$, $R^3$ und X die oben angegebene Bedeutung haben,

mit Säurechloriden der Formel (XIII),

$R^{10}$-Cl (XIII)

in welcher

$R^{10}$ für einen Acyl- oder Sulfonylrest, steht, gegebenenfalls in Gegenwart eines Verdünnungsmittels und gegebenenfalls in Gegenwart eines Säurebindemittels bei Temperaturen zwischen -20° C und +120° C umsetzt.

**Patentansprüche für folgenden Vertragsstaat : ES**

**1.** Verfahren zur Herstellung von substituierten Acrylsäureestern der allgemeinen Formel (I),

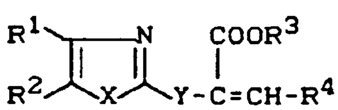

(I)

in welcher

$R^1$ und $R^2$ unabhängig voneinander jeweils für Wasserstoff, für geradkettiges oder verzweigtes Alkyl mit 1 bis 8 Kohlenstoffatomen, für geradkettiges oder verzweigtes Alkenyl mit 2 bis 8 Kohlenstoffatomen, für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder Aryl mit 6 bis 10 Kohlenstoffatomen im jeweiligen Arylteil stehen, wobei als Arylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verchiedenen Halogenatomen substituiertes Heteroarylalkyl oder Heteroaryl mit jeweils 2 bis 9 Kohlenstoffatomen und 1 bis 4 gleichen oder verschiedenen Heteroatomen im Heteroarylteil und gegebenenfalls 1 bis 4 Kohlenstoffatomen im geradkettigen oder verzweigten Alkylteil;

außerdem für einen gegebenenfalls einfach oder mehrfach, gleich oder verschieden substituierten Heteroarylrest mit 2 bis 9 Kohlenstoffatomen und 1 bis 4 gleichen oder verschiedenen Heteroatomen stehen, wobei als Substituenten die oben genannten Arylsubstituenten infrage kommen,

$R^3$ für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ und $R^2$ genannten infrage kommen,

$R^4$ für Dialkylamino mit jeweils 1 bis 6 Kohlenstoffatomen in den einzelnen Alkylteilen steht oder für einen Rest $-Z-R^5$ steht,

X für Sauerstoff oder Schwefel steht und

Y für Sauerstoff, Schwefel oder für einen Rest

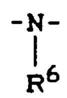

steht,

wobei

$R^5$ für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ und $R^2$ genannten infrage kommen,

$R^6$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil oder Aryl mit jeweils 6 bis 10 Kohlenstoffatomen im jeweiligen Arylteil steht, wobei als Substituenten im Arylteil jeweils die bei $R^1$ und $R^2$ genannten infrage kommen und

Z für Sauerstoff oder Schwefel steht, dadurch gekennzeichnet, daß man

(a) substituierte Acrylsäureester der Formel (Ia),

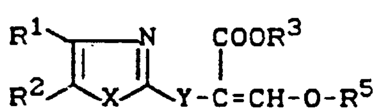

(Ia)

in welcher

$R^1$, $R^2$, $R^3$, $R^5$, X und Y die oben angegebene Bedeutung haben, erhält, wenn man Hydroxyacrylsäureester oder deren Alkalimetallsalze der Formel (II),

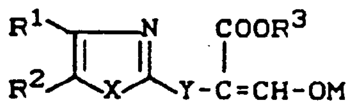 (II)

in welcher

M für Wasserstoff oder für ein Alkalimetallkation steht und

$R^1$, $R^2$, $R^3$, X und Y die oben angegebene Bedeutung haben,

mit Alkylierungsmitteln der Formel (III),

$$R^5\text{-}E^1 \qquad (III)$$

in welcher

$E^1$ für eine elektronenanziehende Abgangsgruppe steht und

$R^5$ die oben angegebene Bedeutung hat,

gegebenenfalls in Gegenwart eines Verdünnungsmittels und gegebenenfalls in Gegenwart eines Reaktionshilfsmittels umsetzt oder

(b) substituierte Acrylsäureester der Formel (Ib),

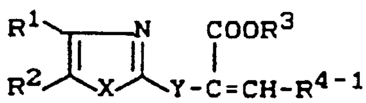 (Ib)

in welcher

$R^{4-1}$ für Dialkylamino steht und

$R^1$, $R^2$, $R^3$, X und Y die oben angegebene Bedeutung haben, erhält,

wenn man substituierte Essigsäureester der Formel (IV),

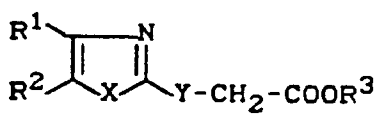 (IV)

in welcher

$R^1$, $R^2$, $R^3$, X und Y die oben angegebene Bedeutung haben,

mit Formamiden der Formel (Va),

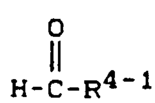 (Va)

in welcher

$R^{4-1}$ die oben angegebene Bedeutung hat,

oder mit deren Derivaten der Formel (Vb),

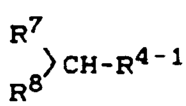 (Vb)

in welcher

$R^7$ und $R^8$ unabhängig voneinander jeweils für Alkoxy oder Dialkylamino stehen und

$R^{4-1}$ die oben angegebene Bedeutung hat,

gegebenenfalls in Gegenwart einem Verdünnungsmittels umsetzt oder

(c) substituierte Acrylsäureester der Formel (Ic),

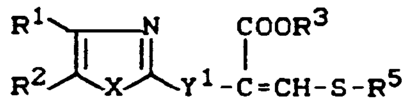 (Ic)

in welcher

$Y^1$ für Schwefel oder für einen Rest

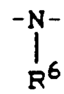

steht,

und

$R^1$, $R^2$, $R^3$, $R^5$, $R^6$ und X die oben angegebene Bedeutung haben, erhält, wenn man Oxalsäurederivate der Formel (VI),

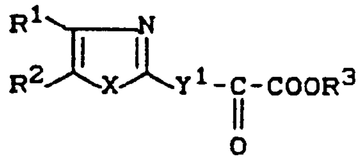 (VI)

in welcher

$R^1$, $R^2$, $R^3$, X und $Y^1$ die oben angegebene Bedeutung haben, mit metallorganischen Verbindungen der Formel (VII),

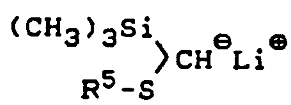 (VII)

in welcher

$R^5$ die oben angegebene Bedeutung hat, gegebenenfalls in Gegenwart eines Verdünnungsmittels umsetzt oder

(d) substituierte Acrylsäureester der Formel (Id),

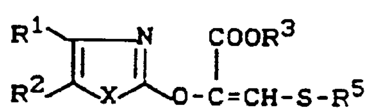 (Id)

in welcher

$R^1$, $R^2$, $R^3$, $R^5$ und X die oben angegebene Bedeutung haben, erhält, wenn man substituierte Acrylsäureester der Formel (VIII),

**2.** Verfahren gemäß Anspruch 1, wobei in der Formel (I)

$R^1$ und $R^2$ unabhängig voneinander jeweils für Wasserstoff, Methyl, Ethyl, n- oder i-Propyl, n-, i-, s-oder t-Butyl, für Allyl, n- oder i-Butenyl , für jeweils gegebenenfalls im Arylteil oder im Heteroarylteil ein- bis dreifach, gleich oder verschieden substituiertes Benzyl, Phenylethyl, Phenylethenyl, Phenyl, Naphthyl, Pyridyl, Thienyl oder Furyl stehen, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio,

Trifluormethyl, Difluormethoxy, Trifluormethoxy und/oder Trifluormethylthio substituiertes Phenyl, Benzyl, Phenoxy oder Benzyloxy,

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen,

$R^4$ für Dialkylamino mit jeweils 1 bis 4 Kohlenstoffatomen in den einzelnen Alkylteilen steht oder für einen Rest -Z-$R^5$ steht,

X für Sauerstoff oder Schwefel steht und

Y für Sauerstoff, Schwefel oder für einen Rest

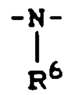

steht,

wobei

$R^5$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen;

$R^6$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ und $R^2$ genannten infrage kommen und

Z für Sauerstoff oder Schwefel steht.

**3.** Verfahren gemäß Anspruch 1, wobei in der Formel

$R^1$ und $R^2$ unabhängig voneinander jeweils für Wasserstoff, Methyl, Ethyl, n- oder i-Propyl oder für jeweils gegebenenfalls ein- bis dreifach, gleich oder verschieden substituiertes Phenyl oder Naphthyl stehen, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n-oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und/oder Ethyl substituiertes Phenyl, Phenoxy, Benzyl oder Benzyloxy,

$R^3$ für Methyl, Ethyl oder Benzyl steht,

$R^4$ für Dimethylamino, Diethylamino oder für einen Rest -Z-$R^5$ steht,

X für Sauerstoff oder Schwefel steht und

Y für Sauerstoff, Schwefel oder für einen Rest

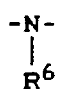

steht,

wobei

$R^5$ für Methyl, Ethyl, n- oder i-Propyl oder Benzyl steht,

$R^6$ für Wasserstoff, Methyl, Ethyl, Acetyl, Propionyl oder für jeweils gegebenenfalls einfach oder zweifach, gleich oder verschieden durch Fluor, Chlor, Brom, Methyl, Ethyl und/ oder Trifluormethyl substituiertes Benzyl oder Phenyl steht und

Z für Sauerstoff oder Schwefel steht.

**4.** Verfahren gemäß Anspruch 1, wobei in der Formel (I)

$R^1$ für Wasserstoff, Methyl, Ethyl, n- oder i-Propyl oder für gegebenenfalls einfach oder zweifach, gleich oder verschieden substituiertes Phenyl steht, wobei als Substituenten infrage kommen:
Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Methoximinoethyl, Cyclopentyl, 1,3-Propandiyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Wasserstoff, Methyl, Ethyl, n- oder i-Propyl steht,

$R^3$ für Methyl oder Ethyl steht,
$R^4$ für Methoxy, Ethox, Methylthio oder Dimethylamino steht,
X für Sauerstoff oder Schwefel steht und
Y für einen N-Methyl-Rest steht.

5. Schädlingsbekämpfungsmittel, gekennzeichnet durch einen Gehalt an mindestens einem substituierten Acrylsäureester der Formel (I) nach Anspruch 1 .

6. Verwendung von substituierten Acrylsäureester der Formel (I) nach Anspruch 1 zur Bekämpfung von Schädlingen.

7. Verfahren zur Bekämpfung von Schädlingen, dadurch gekennzeichnet, daß man substituierte Acrylsäureester der Formel (I) nach Anspruch 1 auf Schädlinge und/oder ihren Lebensraum einwirken läßt.

8. Verfahren zur Herstellung von Schädlingsbekämpfungsmitteln, dadurch gekennzeichnet, daß man substituierte Acrylsäureester der Formel (I) nach Anspruch 1 mit Streckmitteln und/oder oberflächenaktiven Mitteln vermischt.

9. Verfahren zur Herstellung von Hydroxyacrylsäureestern der Formel (IIa),

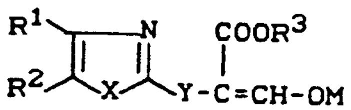

(IIa)

in welcher $R^1$, $R^2$, $R^3$, X und Y die in Anspruch 1 gegebenen Bedeutungen haben, und
M für Wasserstoff oder für ein Alkalimetallkation steht, ausgenommen die Verbindung 2-[4,5-Bis(4-methoxyphenyl)-thiazol-2-yl-thiol-3-hydroxyacrylsäureethylester,
dadurch gekennzeichnet, daß man substituierte Essigsäureester der Formel (IV),

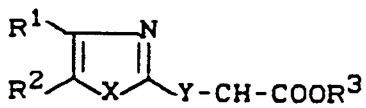

(IV)

in welcher
$R^1$, $R^2$, $R^3$, X und Y die oben angegebene Bedeutung haben,
mit Ameisensäureestern der Formel (X),

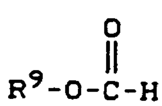

(X)

in welcher
$R^9$ für Alkyl, steht,
gegebenenfalls in Gegenwart eine Verdünnungsmittels und gegebenenfalls in Gegenwart eines basischen Reaktionshilfsmittels bei Temperaturen zwischen -20° C und +50° C umsetzt.

10. Verfahren zur Herstellung von substituierten Acrylsäureestern der Formel (VIII),

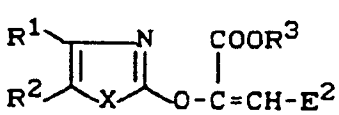

(VIII)

in welcher
$E^2$ für eine elektronenanziehende Abgangsgruppe steht, und

in welcher $R^1$, $R^2$, $R^3$, X die in Anspruch 1 gegebenen Bedeutungen haben, dadurch gekennzeichnet, daß man Hydroxyacrylsäureester der Formel (IIb),

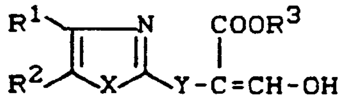

(IIb)

in welcher

$R^1$, $R^2$, $R^3$, Y und X die oben angegebene Bedeutung haben, mit Säurechloriden der Formel (XIII),

$R^{10}$-Cl (XIII)

in welcher

$R^{10}$ für einen Acyl- oder Sulfonylrest, steht,

gegebenenfalls in Gegenwart eines Verdünnungsmittels und gegebenenfalls in Gegenwart eines Säurebindemittels bei Temperaturen zwischen -20°C und +120° C umsetzt.

## Claims

### Claims for the following Contracting States: BE, CH, DE, FR, GB, IT, LI, NL

1. Substituted acrylic acid esters of the general formula (I)

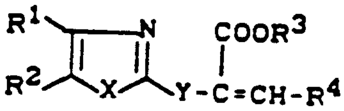

(I)

in which

$R^1$ and $R^2$ independently of one another each represent hydrogen, straight-chain or branched alkyl which has 1 to 8 carbon atoms, straight-chain or branched alkenyl which has 2 to 8 carbon atoms or aralkyl which has 1 to 6 carbon atoms in the straight-chain or branched alkyl moiety, aralkenyl which has 2 to 6 carbon atoms in the straight-chain or branched alkenyl moiety or aryl, each of which has 6 to 10 carbon atoms in the respective aryl moiety and each of which is optionally substituted once or more than once in the aryl moiety by identical or different substituents, suitable aryl substituents in each case being:

halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl which has 3 to 7 carbon atoms, divalent alkanediyl which has 3 to 5 carbon atoms, or aryl, aralkyl, aryloxy or aralkyloxy, each of which has 6 to 10 carbon atoms in the aryl moiety and where appropriate 1 to 4 carbon atoms in the straight-chain or branched alkyl moiety and each of which is optionally substituted once or more than once in the aryl moiety by identical or different substituents from the series comprising halogen, alkyl, alkoxy, alkylthio, halogenoalkyl, halogenoalkoxy and halogenoalkylthio, each of which has 1 to 4 carbon atoms and where appropriate 1 to 9 identical or different halogen atoms, or heteroarylalkyl or heteroaryl, each of which has 2 to 9 carbon atoms and 1 to 4 identical or different hetero atoms in the heteroaryl moiety and where appropriate 1 to 4 carbon atoms in the straight-chain or branched alkyl moiety and each of which is optionally substituted once or more than once in the heteroaryl moiety by identical or different substituents from the series comprising halogen, alkyl, alkoxy, alkylthio, halogenoalkyl, halogenoalkoxy and halogenoalkylthio, each of which has 1 to 4 carbon atoms and where appropriate 1 to 9 identical or different halogen atoms;

furthermore represent a heteroaryl radical which has 2 to 9 carbon atoms and 1 to 4 identical or different hetero atoms, and which is optionally substituted once or more than once by identical or different substituents, suitable substituents being the abovementioned aryl substituents,

$R^3$ represents straight-chain or branched alkyl which has 1 to 6 carbon atoms or aralkyl which has 1 to 4 carbon atoms in the straight-chain or branched alkyl moiety and 6 to 10 carbon atoms in the aryl

moiety and which is optionally substituted once or more than once in the aryl moiety by identical or different substituents, suitable aryl substituents being those which have been mentioned for $R^1$ and $R^2$,

$R^4$ represents dialkylamino, each of which has 1 to 6 carbon atoms in the individual alkyl moieties, or a radical -Z-$R^5$,

X represents oxygen or sulphur and

Y represents oxygen, sulphur or a radical

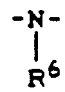 ,

wherein

$R^5$ represents straight-chain or branched alkyl which has 1 to 6 carbon atoms or aralkyl which has 1 to 4 carbon atoms in the straight-chain or branched alkyl moiety and 6 to 10 carbon atoms in the aryl moiety and which is optionally substituted once or more than once in the aryl moiety by identical or different substituents, suitable aryl substituents being those which have been mentioned for $R^1$ and $R^2$,

$R^6$ represents hydrogen, straight-chain or branched alkyl which has 1 to 6 carbon atoms, straight-chain or branched alkanoyl which has 1 to 6 carbon atoms in the alkyl moiety, or aralkyl which has 1 to 6 carbon atoms in the straight-chain or branched alkyl moiety or aryl, each of which has 6 to 10 carbon atoms in the respective aryl moiety and each of which is optionally substituted once or more than once in the aryl moiety by identical or different substituents, suitable substituents in the aryl moiety in each case being those which have been mentioned for $R^1$ and $R^2$, and

Z represents oxygen or sulphur.

**2.** Substituted acrylic acid esters according to Claim 1, wherein, in the formula (I),

$R^1$ and $R^2$ independently of one another each represent hydrogen, methyl, ethyl, n- or i-propyl, n-, i-, s- or t-butyl, allyl, n- or i-butenyl, or benzyl, phenylethyl, phenylethenyl, phenyl, naphthyl, pyridyl, thienyl or furyl, each of which is optionally substituted once, twice or three times in the aryl moiety or in the heteroaryl moiety by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl, or phenyl, benzyl, phenoxy or benzyloxy, each of which is optionally substituted once, twice or three times in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio,

$R^3$ represents methyl, ethyl, n- or i-propyl, n-, i-, s- or t-butyl or benzyl which is optionally substituted once, twice or three times by identical or different substituents, suitable substituents being those which have been mentioned for $R^1$ and $R^2$,

$R^4$ represents dialkylamino which has in each case 1 to 4 carbon atoms in the individual alkyl moieties or a radical -Z-$R^5$,

X represents oxygen or sulphur and

Y represents oxygen, sulphur or a radical

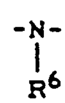 ,

wherein

$R^5$ represents methyl, ethyl, n- or i-propyl, n-, i-, s- or t-butyl or benzyl which is optionally substituted once, twice or three times by identical or different substituents, suitable substituents being those which have been mentioned for $R^1$ and $R^2$;

$R^6$ represents hydrogen, methyl, ethyl, n- or i-propyl, n-, i-, s- or t-butyl, acetyl, propionyl, n- or i-butyryl, or benzyl or phenyl, each of which is optionally substituted once, twice or three times by identical or different substituents, suitable substituents being those which have been mentioned for $R^1$ and $R^2$ and

Z represents oxygen or sulphur.

3. Substituted acrylic acid esters according to Claim 1, wherein, in the formula,
$R^1$ and $R^2$ independently of one another each represent hydrogen, methyl, ethyl, n- or i-propyl, or phenyl or naphthyl, each of which is optionally substituted once, twice or three times by identical or different substituents, suitable substituents in each case being: fluorine, chlorine, bromine, cyano, nitro, methyl, ethyl, n- or i-propyl, n-, i-, s- or t-butyl methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl, or phenyl, phenoxy, benzyl or benzyloxy, each of which is optionally substituted once or twice by identical or different substituents from the series comprising fluorine, chlorine, bromine, methyl and/or ethyl,
$R^3$ represents methyl, ethyl or benzyl,
$R^4$ represents dimethylamino, diethylamino or a radical -Z-$R^5$,
X represents oxygen or sulphur and
Y represents oxygen, sulphur or a radical

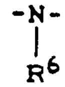 ,

wherein
$R^5$ represents methyl, ethyl, n- or i-propyl or benzyl,
$R^6$ represents hydrogen, methyl, ethyl, acetyl, propionyl, or benzyl or phenyl, each of which is optionally substituted once or twice by identical or different substituents from the series comprising fluorine, chlorine, bromine, methyl, ethyl and/or trifluoromethyl, and
Z represents oxygen or sulphur.

4. Substituted acrylic acid esters according to Claim 1, wherein, in the formula (I),
$R^1$ represents hydrogen, methyl, ethyl, n- or i-propyl, or phenyl which is optionally substituted once or twice by identical or different substituents, suitable substituents being:
fluorine, chlorine, bromine, cyano, nitro, methyl, ethyl, methoxy, ethoxy, methylthio, trifluoromethyl, trifluoromethoxy, trifluoromethylthio, methoxycarbonyl, ethoxycarbonyl, methoximinomethyl, methoximinoethyl, cyclopentyl, 1,3-propanediyl, or phenyl, phenoxy, benzyl or benzyloxy, each of which is optionally substituted once or twice by identical or different substituents from the series comprising fluorine, chlorine, bromine and methyl,
$R^2$ represents hydrogen, methyl, ethyl, n- or i-propyl,
$R^3$ represents methyl or ethyl,
$R^4$ represents methoxy, ethoxy, methylthio or dimethylamino,
X represents oxygen or sulphur and
Y represents an N-methyl radical.

5. Process for the preparation of substituted acrylic acid esters of the general formula (I)

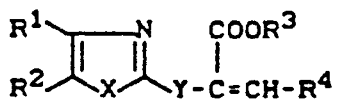 (I)

in which $R^1$, $R^2$, $R^3$, $R^4$, X and Y have the meanings indicated in Claim 1,
characterised in that
(a) substituted acrylic acid esters of the formula (Ia)

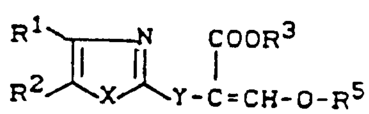 (Ia)

in which

$R^1$, $R^2$, $R^3$, $R^5$, X and Y have the abovementioned meaning,

are obtained when hydroxyacrylic acid esters or their alkali metal salts of the formula (II)

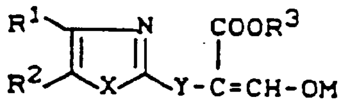

(II)

in which

M represents hydrogen or an alkali metal cation and

$R^1$, $R^2$, $R^3$, X and Y have the abovementioned meaning

are reacted with alkylating agents of the formula (III)

$R^5$-$E^1$ (III)

in which

$E^1$ represents an electron-withdrawing leaving group and

$R^5$ has the abovementioned meaning,

if appropriate in the presence of a diluent and if appropriate in the presence of a reaction auxiliary

or

(b) substituted acrylic acid esters of the formula (Ib)

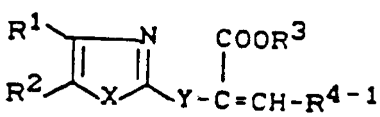

(Ib)

in which

$R^{4-1}$ represents dialkylamino and

$R^1$, $R^2$, $R^3$, X and Y have the abovementioned meaning

are obtained when substituted acetic acid esters of the formula (IV)

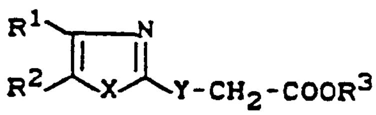

(IV)

in which

$R^1$, $R^2$, $R^3$, X and Y have the abovementioned meaning

are reacted with formamides of the formula (Va)

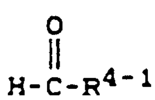

(Va)

in which

$R^{4-1}$ has the abovementioned meaning

or with derivatives thereof of the formula (Vb)

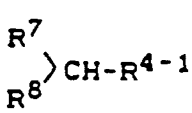

(Vb)

in which

$R^7$ and $R^8$ independently of one another each represent alkoxy or dialkylamino and

$R^{4-1}$ has the abovementioned meaning,

if appropriate in the presence of a diluent or

(c) substituted acrylic acid esters of the formula (Ic)

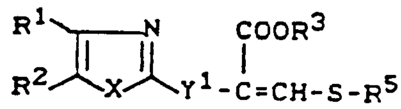 (Ic)

in which

Y[1] represents sulphur or a radical

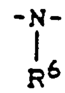

and

$R^1$, $R^2$, $R^3$, $R^5$, $R^6$ and X have the abovementioned meaning are obtained when oxalic acid derivatives of the formula (VI)

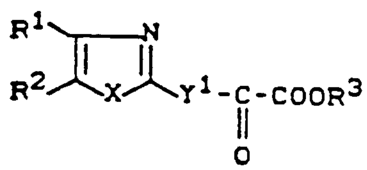 (VI)

in which

$R^1$, $R^2$, $R^3$, X and $Y^1$ have the abovementioned meaning are reacted with organometal compounds of the formula (VII)

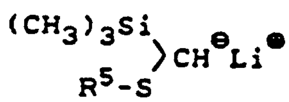 (VII)

in which

$R^5$ has the abovementioned meaning, if appropriate in the presence of a diluent or

(d) substituted acrylic acid esters of the formula (Id)

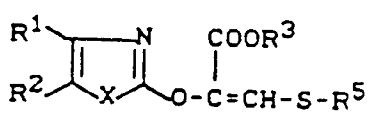 (Id)

in which

$R^1$, $R^2$, $R^3$, $R^5$ and X have the abovementioned meaning are obtained when substituted acrylic acid esters of the formula (VIII)

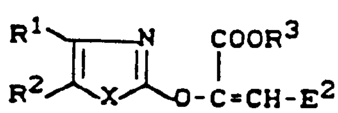 (VIII)

in which

$E^2$ represents an electron-withdrawing leaving group and

$R^1$, $R^2$, $R^3$ and X have the abovementioned meaning are reacted with thiols of the formula (IX)

$R^5$-SH (IX)

in which

$R^5$ has the abovementioned meaning,

if appropriate in the presence of a diluent and if appropriate in the presence of a reaction auxiliary.

**6.** Pesticide, characterised in that it contains at least one substituted acrylic acid ester of the formula (I) according to Claims 1 or 5.

**7.** Use of substituted acrylic acid esters of the formula (I) according to Claims 1 or 5 for combating pests.

**8.** Method of combating pests, characterised in that substituted acrylic acid esters of the formula (I) according to Claims 1 or 5 are allowed to act on pests and/or their habitat.

**9.** Process for the preparation of pesticides, characterised in that substituted acrylic acid esters of the formula (I) according to Claims 1 or 5 are mixed with extenders and/or surface-active substances.

**10.** Hydroxyacrylic acid esters of the formula (IIa)

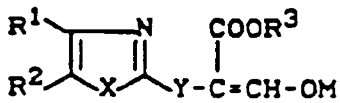

(IIa)

in which $R^1$, $R^2$, $R^3$, X and Y have the meanings indicated in Claim 1, and

M represents hydrogen or an alkali metal cation, with the exception of the compound ethyl 2-[4,5-bis(4-methoxyphenyl)-thiazol-2-yl-thio]-3-hydroxyacrylate.

**11.** Process for the preparation of hydroxyacrylic acid esters of the formula (IIa)

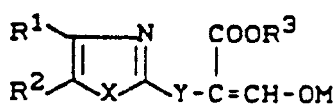

(IIa)

in which

$R^1$, $R^2$, $R^3$, M, X and Y have the meanings indicated in Claim 10,

characterised in that substituted acetic acid esters of the formula (IV)

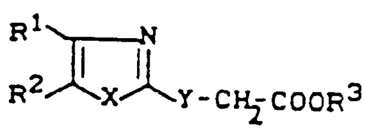

(IV)

in which

$R^1$, $R^2$, $R^3$, X and Y have the abovementioned meaning

are reacted with formic acid esters of the formula (X)

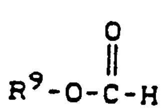

(X)

in which

$R^9$ represents alkyl,

if appropriate in the presence of a diluent and if appropriate in the presence of a basic reaction auxiliary, at temperatures between -20°C and +50°C.

**12.** Substituted acrylic acid esters of the formula (VIII)

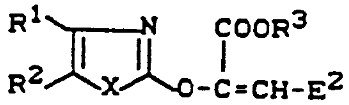

(VIII)

in which $R^1$, $R^2$, $R^3$ and X have the meanings indicated in Claim 1, and
$E^2$ represents an electron-withdrawing leaving group.

13. Process for the preparation of substituted acrylic acid esters of the formula (VIII)

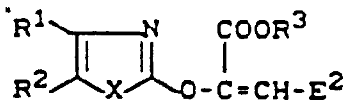

(VIII)

in which $R^1$, $R^2$, $R^3$, X and $E^2$ have the meanings indicated in Claim 12, characterised in that hydroxyacrylic acid esters of the formula (IIb)

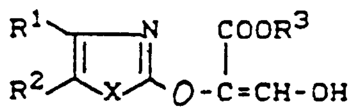

(IIb)

in which
$R^1$, $R^2$, $R^3$ and X have the abovementioned meaning
are reacted with acid chlorides of the formula (XIII)

$R^{10}$-Cl (XIII)

in which
$R^{10}$ represents an acyl or sulphonyl radical,
if appropriate in the presence of a diluent and if appropriate in the presence of an acid-binding agent, at temperatures between -20°C and +120°C.

**Claims for the following Contracting State : ES**

1. Process for the preparation of substituted acrylic acid esters of the general formula (I)

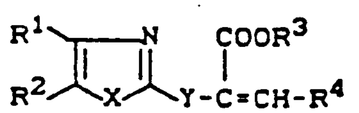

(I)

in which
$R^1$ and $R^2$ independently of one another each represent hydrogen, straight-chain or branched alkyl which has 1 to 8 carbon atoms, straight-chain or branched alkenyl which has 2 to 8 carbon atoms, or aralkyl which has 1 to 6 carbon atoms in the straight-chain or branched alkyl moiety, aralkenyl which has 2 to 6 carbon atoms in the straight-chain or branched alkenyl moiety or aryl, each of which has 6 to 10 carbon atoms in the respective aryl moiety and each of which is optionally substituted once or more than once in the aryl moiety by identical or different substituents, suitable aryl substituents in each case being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl which has 3 to 7 carbon atoms, divalent alkanediyl which has 3 to 5 carbon atoms, or aryl, aralkyl, aryloxy or aralkyloxy, each of which has 6 to 10 carbon atoms in the aryl moiety and where appropriate 1 to 4 carbon atoms in the straight-chain or branched alkyl moiety and each of which is optionally substituted once or more than once in the aryl moiety by identical or different substituents from the series comprising halogen, alkyl, alkoxy, alkylthio, halogenoalkyl, halogenoalkoxy

and halogenoalkylthio, each of which has 1 to 4 carbon atoms and where appropriate 1 to 9 identical or different halogen atoms, or heteroarylalkyl or heteroaryl, each of which has 2 to 9 carbon atoms and 1 to 4 identical or different hetero atoms in the heteroaryl moiety and where appropriate 1 to 4 carbon atoms in the straight-chain or branched alkyl moiety and each of which is optionally substituted once or more than once in the heteroaryl moiety by identical or different substituents from the series comprising halogen, alkyl, alkoxy, alkylthio, halogenoalkyl, halogenoalkoxy and halogenoalkylthio, each of which has 1 to 4 carbon atoms and where appropriate 1 to 9 identical or different halogen atoms;
furthermore represent a heteroaryl radical which has 2 to 9 carbon atoms and 1 to 4 identical or different hetero atoms, and which is optionally substituted once or more than once by identical or different substituents, suitable substituents being the abovementioned aryl substituents,

$R^3$ represents straight-chain or branched alkyl which has 1 to 6 carbon atoms or aralkyl which has 1 to 4 carbon atoms in the straight-chain or branched alkyl moiety and 6 to 10 carbon atoms in the aryl moiety and which is optionally substituted once or more than once in the aryl moiety by identical or different substituents, suitable aryl substituents being those which have been mentioned for $R^1$ and $R^2$,

$R^4$ represents dialkylamino, each of which has 1 to 6 carbon atoms in the individual alkyl moieties, or a radical -Z-$R^5$,

X represents oxygen or sulphur and

Y represents oxygen, sulphur or a radical

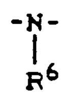 ,

wherein

$R^5$ represents straight-chain or branched alkyl which has 1 to 6 carbon atoms or aralkyl which has 1 to 4 carbon atoms in the straight-chain or branched alkyl moiety and 6 to 10 carbon atoms in the aryl moiety and which is optionally substituted once or more than once in the aryl moiety by identical or different substituents, suitable aryl substituents being those which have been mentioned for $R^1$ and $R^2$,

$R^6$ represents hydrogen, straight-chain or branched alkyl which has 1 to 6 carbon atoms, straight-chain or branched alkanoyl which has 1 to 6 carbon atoms in the alkyl moiety, or aralkyl which has 1 to 6 carbon atoms in the straight-chain or branched alkyl moiety or aryl, each of which has 6 to 10 carbon atoms in the respective aryl moiety and each of which is optionally substituted once or more than once in the aryl moiety by identical or different substituents, suitable substituents in the aryl moiety in each case being those which have been mentioned for $R^1$ and $R^2$, and

Z represents oxygen or sulphur,

characterised in that

(a) substituted acrylic acid esters of the formula (Ia)

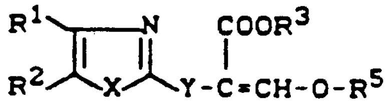

(Ia)

in which

$R^1$, $R^2$, $R^3$, $R^5$, X and Y have the abovementioned meaning,

are obtained when hydroxyacrylic acid esters or their alkali metal salts of the formula (II)

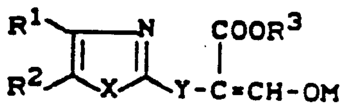

(II)

in which

M represents hydrogen or an alkali metal cation and

$R^1$, $R^2$, $R^3$, X and Y have the abovementioned meaning

are reacted with alkylating agents of the formula (III)

$R^5$-$E^1$ (III)

in which

$E^1$ represents an electron-withdrawing leaving group and

$R^5$ has the abovementioned meaning,

if appropriate in the presence of a diluent and if appropriate in the presence of a reaction auxiliary

or

(b) substituted acrylic acid esters of the formula (Ib)

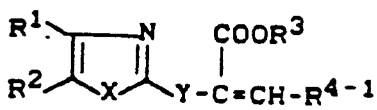

(Ib)

in which

$R^{4-1}$ represents dialkylamino and

$R^1$, $R^2$, $R^3$, X and Y have the abovementioned meaning are obtained when substituted acetic acid esters of the formula (IV)

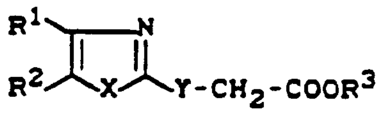

(IV)

in which

$R^1$, $R^2$, $R^3$, X and Y have the abovementioned meaning

are reacted with formamides of the formula (Va)

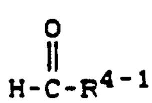

(Va)

in which

$R^{4-1}$ has the abovementioned meaning

or with derivatives thereof of the formula (Vb)

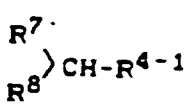

(Vb)

in which

$R^7$ and $R^8$ independently of one another each represent alkoxy or dialkylamino and

$R^{4-1}$ has the abovementioned meaning,

if appropriate in the presence of a diluent or

(c) substituted acrylic acid esters of the formula (Ic)

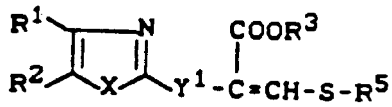

(Ic)

in which

$Y^1$ represents sulphur or a radical

and

$R^1$, $R^2$, $R^3$, $R^5$, $R^6$ and X have the abovementioned meaning

are obtained when oxalic acid derivatives of the formula (VI)

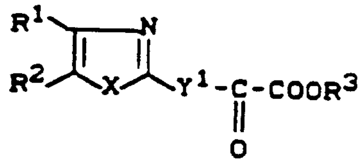

(VI)

in which

$R^1$, $R^2$, $R^3$, X and $Y^1$ have the abovementioned meaning

are reacted with organometal compounds of the formula (VII)

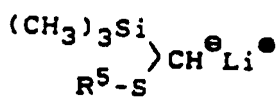

(VII)

in which

$R^5$ has the abovementioned meaning,

if appropriate in the presence of a diluent or

(d) substituted acrylic acid esters of the formula (Id)

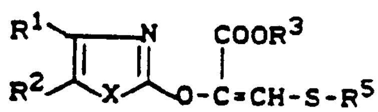

(Id)

in which

$R^1$, $R^2$, $R^3$, $R^5$ and X have the abovementioned meaning

are obtained when substituted acrylic acid esters of the formula (VIII)

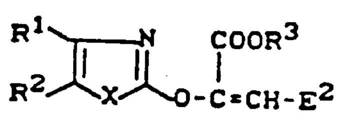

(VIII)

in which

$E^2$ represents an electron-withdrawing leaving group and

$R^1$, $R^2$, $R^3$ and X have the abovementioned meaning

are reacted with thiols of the formula (IX)

$$R^5\text{-SH} \qquad \text{(IX)}$$

in which

$R^5$ has the abovementioned meaning,

if appropriate in the presence of a diluent and if appropriate in the presence of a reaction auxiliary.

2. Process according to Claim 1, wherein, in the formula (I),

$R^1$ and $R^2$ independently of one another each represent hydrogen, methyl, ethyl, n- or i-propyl, n-, i-, s- or t-butyl, allyl, n- or i-butenyl, or benzyl, phenylethyl, phenylethenyl, phenyl, naphthyl, pyridyl, thienyl or furyl, each of which is optionally substituted once, twice or three times in the aryl moiety or in the heteroaryl moiety by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl, or phenyl, benzyl, phenoxy or benzyloxy, each of which is optionally substituted once, twice or three times in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio,

$R^3$ represents methyl, ethyl, n- or i-propyl, n-, i-, s- or t-butyl or benzyl which is optionally substituted once, twice or three times by identical or different substituents, suitable substituents being those which have been mentioned for $R^1$ and $R^2$,

$R^4$ represents dialkylamino which has in each case 1 to 4 carbon atoms in the individual alkyl moieties or a radical -Z-$R^5$,

X represents oxygen or sulphur and

Y represents oxygen, sulphur or a radical

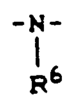 ,

wherein

$R^5$ represents methyl, ethyl, n- or i-propyl, n-, i-, s- or t-butyl or benzyl which is optionally substituted once, twice or three times by identical or different substituents, suitable substituents being those which have been mentioned for $R^1$ and $R^2$;

$R^5$ represents hydrogen, methyl, ethyl, n- or i-propyl, n-, i-, s- or t-butyl, acetyl, propionyl, n- or i-butyryl, or benzyl or phenyl, each of which is optionally substituted once, twice or three times by identical or different substituents, suitable substituents being those which have been mentioned for $R^1$ and $R^2$ and

Z represents oxygen or sulphur.

3. Process according to Claim 1, wherein, in the formula,

$R^1$ and $R^2$ independently of one another each represent hydrogen, methyl, ethyl, n- or i-propyl, or phenyl or naphthyl, each of which is optionally substituted once, twice or three times by identical or different substituents, suitable substituents in each case being: fluorine, chlorine, bromine, cyano, nitro, methyl, ethyl, n- or i -propyl, n-, i -, s - or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl, or phenyl, phenoxy, benzyl or benzyloxy, each of which is optionally substituted once or twice by identical or different substituents from the series comprising fluorine, chlorine, bromine, methyl and/or ethyl,

$R^3$ represents methyl, ethyl or benzyl,

$R^4$ represents dimethylamino, diethylamino or a radical -Z -$R^5$,

X represents oxygen or sulphur and

Y represents oxygen, sulphur or a radical

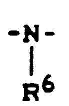 ,

wherein

$R^5$ represents methyl, ethyl, n- or i-propyl or benzyl,

$R^6$ represents hydrogen, methyl, ethyl, acetyl, propionyl, or benzyl or phenyl, each of which is optionally substituted once or twice by identical or different substituents from the series comprising fluorine, chlorine, bromine, methyl, ethyl and/or trifluoromethyl, and

Z represents oxygen or sulphur.

4. Process according to Claim 1, wherein, in the formula (I),

$R^1$ represents hydrogen, methyl, ethyl, n- or i-propyl, or phenyl which is optionally substituted once or twice by identical or different substituents, suitable substituents being:
fluorine, chlorine, bromine, cyano, nitro, methyl, ethyl, methoxy, ethoxy, methylthio, trifluoromethyl, trifluoromethoxy, trifluoromethylthio, methoxycarbonyl, ethoxycarbonyl, methoximinomethyl, methoximinoethyl, cyclopentyl, 1,3-propanediyl, or phenyl, phenoxy, benzyl or benzyloxy, each of which is optionally substituted once or twice by identical or different substituents from the series comprising fluorine, chlorine, bromine and methyl,

$R^2$ represents hydrogen, methyl, ethyl, n- or i-propyl,
$R^3$ represents methyl or ethyl,
$R^4$ represents methoxy, ethoxy, methylthio or dimethylamino,
X represents oxygen or sulphur and
Y represents an N-methyl radical.

5. Pesticide, characterised in that it contains at least one substituted acrylic acid ester of the formula (I) according to Claim 1.

6. Use of substituted acrylic acid esters of the formula (I) according to Claim 1 for combating pests.

7. Method of combating pests, characterised in that substituted acrylic acid esters of the formula (I) according to Claim 1 are allowed to act on pests and/or their habitat.

8. Process for the preparation of pesticides, characterised in that substituted acrylic acid esters of the formula (I) according to Claim 1 are mixed with extenders and/or surface-active substances.

9. Process for the preparation of hydroxyacrylic acid esters of the formula (IIa)

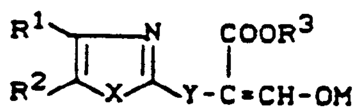

(IIa)

in which $R^1$, $R^2$, $R^3$, X and Y have the meanings indicated in Claim 1, and

M represents hydrogen or an alkali metal cation, with the exception of the compound ethyl 2-[4,5-bis(4-methoxyphenyl)-thiazol-2-yl-thio]-3-hydroxyacrylate,

characterised in that substituted acetic acid esters of the formula (IV)

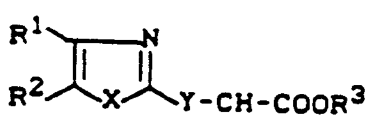

(IV)

in which

$R^1$, $R^2$, $R^3$, X and Y have the abovementioned meaning
are reacted with formic acid esters of the formula (X)

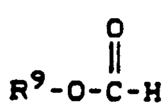

(X)

in which

$R^9$ represents alkyl,
if appropriate in the presence of a diluent and if appropriate in the presence of a basic reaction

auxiliary, at temperatures between -20°C and +50°C.

10. Process for the preparation of substituted acrylic acid esters of the formula (VIII)

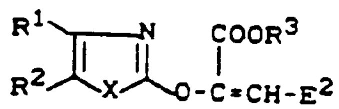

(VIII)

in which

E² represents an electron-withdrawing leaving group, and in which $R^1$, $R^2$, $R^3$ and X have the meanings given in Claim 1, characterised in that hydroxyacrylic acid esters of the formula (IIb)

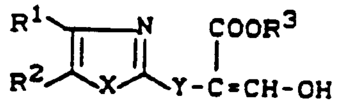

(IIb)

in which

$R^1$, $R^2$, $R^3$, Y and X have the abovementioned meaning are reacted with acid chlorides of the formula (XIII)

$R^{10}$-Cl (XIII)

in which

$R^{10}$ represents an acyl or sulphonyl radical,

if appropriate in the presence of a diluent and if appropriate in the presence of an acid-binding agent, at temperatures between -20°C and +120°C.

## Revendications

### Revendications pour les Etats contractants suivants : BE, CH, DE, FR, GB, IT, LI, NL

1. Esters acryliques substitués répondant à la formule générale (I)

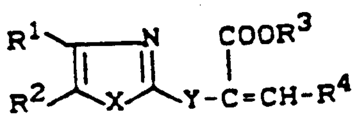

(I)

dans laquelle

$R^1$ et $R^2$ représentent chacun, indépendamment l'un de l'autre, un atome d'hydrogène, un groupe alkyle à chaîne droite ou ramifiée contenant de 1 à 8 atomes de carbone, un groupe alcényle à chaîne droite ou ramifiée contenant de 2 à 8 atomes de carbone, un groupe aralkyle contenant de 1 à 6 atomes de carbone dans la fraction alkyle à chaîne droite ou ramifiée, un groupe aralcényle contenant de 2 à 6 atomes de carbone dans la fraction alcényle à chaîne droite ou ramifiée ou un groupe aryle contenant de 6 à 10 atomes de carbone dans chaque fraction aryle, ces groupes étant chacun éventuellement substitués une ou plusieurs fois de manière identique ou différente dans la fraction aryle, dans lesquels, comme substituants du groupe aryle, entrent chaque fois en ligne de compte : un atome d'halogène, un groupe cyano, un groupe nitro, un groupe alkyle, un groupe alcoxy ou un groupe alkylthio contenant chacun de 1 à 4 atomes de carbone et étant chacun à chaîne droite ou ramifiée, un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée, un groupe alcoxycarbonyle ou un groupe alcoximinoalkyle contenant chacun de 1 à 8 atomes de carbone dans les fractions alkyle individuelles, chacun de ces groupes étant à chaîne droite ou ramifiée, un groupe cycloalkyle contenant de 3 à 7 atomes de carbone, un groupe alcanediyle lié deux fois contenant de 3 à 5 atomes de carbone, un groupe aryle, un groupe aralkyle, un groupe aryloxy ou un groupe aralkyloxy contenant chacun de 6 à 10 atomes de carbone dans la fraction aryle et éventuellement de 1 à 4 atomes

de carbone dans la fraction alkyle à chaîne droite ou ramifiée, chacun de ces groupes étant éventuellement substitués une ou plusieurs fois de manière identique ou différente dans la fraction aryle par un atome d'halogène, un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, ou encore un groupe hétéroarylalkyle ou un groupe hétéroaryle contenant chacun de 2 à 9 atomes de carbone et de 1 à 4 hétéro-atomes identiques ou différents dans la fraction hétéroaryle et éventuellement de 1 à 4 atomes de carbone dans la fraction alkyle à chaîne droite ou ramifiée, chacun de ces groupes étant éventuellement substitué une ou plusieurs fois de manière identique ou différente dans la fraction hétéroaryle par un atome d'halogène, un groupe alkyle, un groupe alcoxy, un groupe alkylthio, un groupe halogénalkyle, un groupe halogénalcoxy, ou un groupe halogénalkylthio contenant chacun de 1 à 4 atomes de carbone et éventuellement de 1 à 9 atomes d'halogène identiques ou différents; en outre, un radical hétéroaryle contenant de 2 à 9 atomes de carbone et de 1 à 4 hétéro-atomes identiques ou différents éventuellement substitué une ou plusieurs fois de manière identique ou différente, dans lequel entrent en ligne de compte comme substituants, les substituants mentionnés ci-dessus pour le groupe aryle,

$R^3$ représente un groupe alkyle à chaîne droite ou ramifiée contenant de 1 à 6 atomes de carbone ou un groupe aralkyle contenant de 1 à 4 atomes de carbone dans la fraction alkyle à chaîne droite ou ramifiée et de 6 à 10 atomes de carbone dans la fraction aryle, ce groupe étant éventuellement substitué une ou plusieurs fois de manière identique ou différente dans la fraction aryle, dans lequel comme substituants du groupe aryle, entrent en ligne de compte ceux mentionnés pour $R^1$ et $R^2$,

$R^4$ représente un groupe dialkylamino contenant chaque fois de 1 à 6 atomes de carbone dans les fractions alkyle individuelles ou bien un radical $-Z-R^5$,

X représente un atome d'oxygène ou un atome de soufre et

Y représente un atome d'oxygène, un atome de soufre ou un radical

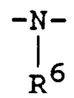

dans lesquels

$R^5$ représente un groupe alkyle à chaîne droite ou ramifiée contenant de 1 à 6 atomes de carbone ou un groupe aralkyle contenant de 1 à 4 atomes de carbone dans la fraction alkyle à chaîne droite ou ramifiée et de 6 à 10 atomes de carbone dans la fraction aryle, ce groupe étant éventuellement substitué une ou plusieurs fois de manière identique ou différente dans la fraction aryle, dans lequel entrent en ligne de compte comme substituants du groupe aryle, ceux mentionnés pour $R^1$ et $R^2$,

$R^6$ représente un atome d'hydrogène, un groupe alkyle à chaîne droite ou ramifiée contenant de 1 à 6 atomes de carbone, un groupe alcanoyle à chaîne droite ou ramifiée contenant de 1 à 6 atomes de carbone dans la fraction alkyle ou un groupe aralkyle contenant de 1 à 6 atomes de carbone dans la fraction alkyle à chaîne droite ou ramifiée, ou encore un groupe aryle contenant chaque fois de 6 à 10 atomes de carbone dans chaque fraction aryle, chacun de ces groupes étant éventuellement substitué une ou plusieurs fois de manière identique ou différente dans la fraction aryle, dans lequel entrent en ligne de compte comme substituants dans la fraction aryle, chaque fois ceux mentionnés pour $R^1$ et $R^2$, et

Z représente un atome d'oxygène ou un atome de soufre.

2. Esters acryliques substitués selon la revendication 1, dans lesquels, dans la formule (I),

$R^1$ et $R^2$ représentent chacun, indépendamment l'un de l'autre, un atome d'hydrogène, un groupe méthyle, un groupe éthyle, un groupe n- ou i-propyle, un groupe n-, i-, s- ou t-butyle, un groupe allyle, un groupe n- ou i-butényle, un groupe benzyle, un groupe phényléthyle, un groupe phényléthényle, un groupe phényle, un groupe naphtyle, un groupe pyridyle, un groupe thiényle ou un groupe furyle, chacun de ces groupes étant éventuellement substitué de 1 à 3 fois de manière identique ou différente dans la fraction aryle ou dans la fraction hétéroaryle, dans lesquels entrent chaque fois en ligne de compte comme substituants : un atome de fluor, un atome de chlore, un atome de brome, un groupe cyano, un groupe nitro, un groupe méthyle, un groupe éthyle, un groupe n- ou i propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe difluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle, un groupe éthoximinoéthyle, un groupe cyclopentyle, un groupe cyclohexyle, un groupe 1,3-propanediyle, un groupe 1,4-butanediyle ou encore un groupe phényle, un groupe benzyle, un groupe phénoxy ou un groupe benzyloxy, chacun de ces groupes étant éventuellement substitué de 1 à 3 fois de manière identique ou différente dans la fraction phényle par un atome de fluor, un atome de chlore, un atome de brome, un groupe méthyle, un groupe éthyle, un groupe méthoxy, un groupe éthoxy, un groupe méthylthio, un groupe trifluorométhyle, un groupe difluorométhoxy, un groupe trifluorométhoxy et/ou un groupe trifluorométhylthio,

$R^3$ représente un groupe méthyle, un groupe éthyle, un groupe n- ou i-propyle, un groupe n-, i-, s- ou t-butyle ou encore un groupe benzyle éventuellement substitué de 1 à 3 fois de manière identique ou différente, dans lequel entrent en ligne de compte comme substituants, ceux mentionnés pour $R^1$ et $R^2$,

$R^4$ représente un groupe dialkylamino contenant chaque fois de 1 à 4 atomes de carbone dans les fractions alkyle individuelles ou encore un radical -Z-$R^5$,

X représente un atome d'oxygène ou un atome de soufre, et

Y représente un atome d'oxygène, un atome de soufre ou un radical

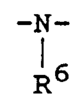

dans lesquels ,

$R^5$ représente un groupe méthyle, un groupe éthyle, un groupe n- ou i-propyle, un groupe n-, i-, s- ou t-butyle ou encore un groupe benzyle éventuellement substitué de 1 à 3 fois de manière identique ou différente, dans lequel entrent en ligne de compte comme substituants ceux mentionnés pour $R^1$ et $R^2$;

$R^6$ représente un atome d'hydrogène, un groupe méthyle, un groupe éthyle, un groupe n- ou i-propyle, un groupe n-, i-, s- ou t-butyle, un groupe acétyle, un groupe propionyle, un groupe n- ou i-butyryle ou encore un groupe benzyle ou un groupe phényle, chacun de ces groupes étant éventuellement substitué de 1 à 3 fois de manière identique ou différente, dans lequel entrent en ligne de compte comme substituants, ceux mentionnés pour $R^1$ et $R^2$, et

Z représente un atome d'oxygène ou un atome de soufre.

**3.** Esters acryliques substitués selon la revendication 1, dans lesquels, dans la formule,

$R^1$ et $R^2$ représentent chacun, indépendamment l'un de l'autre, un atome d'hydrogène, un groupe méthyle, un groupe éthyle, un groupe n- ou i-propyle, ou encore un groupe phényle ou un groupe naphtyle, chacun de ces groupes étant éventuellement substitué de 1 à 3 fois de manière identique ou différente, dans lequel entrent chaque fois en ligne de compte comme substituants :

un atome de fluor, un atome de chlore, un atome de brome, un groupe cyano, un groupe nitro, un groupe méthyle, un groupe éthyle,un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe difluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle, un groupe éthoximinoéthyle, un groupe cyclopentyle, un groupe cyclohexyle, un groupe 1,3-propanediyle, un groupe 1,4-butanediyle ou encore,un groupe phényle, un groupe phénoxy, un groupe benzyle ou un groupe benzyloxy, chacun de ces groupes étant éventuellement substitué 1 ou 2 fois de manière identique ou différente par un atome de fluor, un atome de chlore, un atome de brome, un groupe méthyle et/ou un groupe éthyle,

$R^3$ représente un groupe méthyle, un groupe éthyle ou un groupe benzyle,

$R^4$ représente un groupe diméthylamino, un groupe diéthylamino ou un radical -Z-$R^5$,

X représente un atome d'oxygène ou un atome de soufre, et

Y représente un atome d'oxygène, un atome de soufre ou un radical

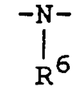

dans lesquels

$R^5$ représente un groupe méthyle, un groupe éthyle, un groupe n- ou i-propyle, ou un groupe benzyle,

$R^6$ représente un atome d'hydrogène, un groupe méthyle, un groupe éthyle, un groupe acétyle, un

groupe propionyle ou encore un groupe benzyle ou un groupe phényle, chacun de ces groupes étant éventuellement substitué 1 ou 2 fois de manière identique ou différente par un atome de fluor, un atome de chlore, un atome de brome, un groupe méthyle, un groupe éthyle et/ou un groupe trifluorométhyle, et

Z représente un atome d'oxygène ou un atome de soufre.

**4.** Esters acryliques substitués selon la revendication 1, dans lesquels, dans la formule (I),

$R^1$ représente un atome d'hydrogène, un groupe méthyle, un groupe éthyle, un groupe n- ou i-propyle ou encore un groupe phényle éventuellement substitué une ou deux fois de manière identique ou différente, dans lequel entrent en ligne de compte comme substituants :
un atome de fluor, un atome de chlore, un atome de brome, un groupe cyano, un groupe nitro, un groupe méthyle, un groupe éthyle, un groupe méthoxy, un groupe éthoxy, un groupe méthylthio, un groupe trifluorométhyle, un groupe trifluorométhoxy, un groupe trifluorométhylthio, un groupe méthoxycarbonyle, un groupe éthoxycarbonyle, un groupe méthoximinométhyle, un groupe méthoximinoéthyle, un groupe cyclopentyle, un groupe 1,3-propanediyle ou encore un groupe phényle, un groupe phénoxy, un groupe benzyle ou un groupe benzyloxy éventuellement substitués une ou deux fois de manière identique ou différente par un atome de fluor, un atome de chlore, un atome de brome ou un groupe méthyle,

$R^2$ représente un atome d'hydrogène, un groupe méthyle, un groupe éthyle, un groupe n- ou i-propyle,

$R^3$ représente un groupe méthyle ou un groupe éthyle,

$R^4$ représente un groupe méthoxy, un groupe éthoxy, un groupe méthylthio ou un groupe diméthylamino,

X représente un atome d'oxygène ou un atome de soufre, et

Y représente un radical N-méthyle.

**5.** Procédé pour la préparation d'esters acryliques substitués répondant à la formule générale (I),

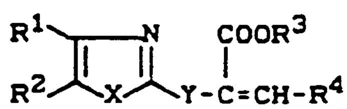 (I)

dans laquelle

$R^1$, $R^2$, $R^3$, $R^4$, X et Y ont les significations mentionnées dans la revendication 1,

**caractérisé en ce qu**'on obtient

(a) des esters acryliques substitués de formule (Ia)

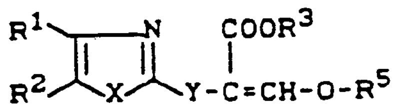 (Ia)

dans laquelle

$R^1$, $R^2$, $R^3$, $R^5$, X et Y ont la signification mentionnée ci-dessus,

lorsqu'on fait réagir des esters hydroxyacryliques ou leurs sels de métaux alcalins de formule (II)

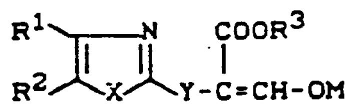 (II)

dans laquelle

M représente un atome d'hydrogène ou un cation de métal alcalin et

$R^1$, $R^2$, $R^3$, X et Y ont la signification mentionnée ci-dessus,

avec des agents d'alkylation de formule (III),

$$R^5\text{-}E^1 \qquad \text{(III)}$$

dans laquelle

$E^1$ représente un groupe qui se sépare, attirant les électrons et

$R^5$ a la signification mentionnée ci-dessus,

éventuellement en présence d'un diluant et éventuellement en présence d'un adjuvant réactionnel; ou bien on obtient

(b)des esters acryliques substitués de formule (Ib),

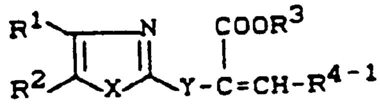

(Ib)

dans laquelle

$R^{4-1}$ représente un groupe dialkylamino et

$R^1$, $R^2$, $R^3$, X et Y ont la signification mentionnée ci-dessus,

lorsqu'on fait réagir des esters acétiques substitués de formule (IV),

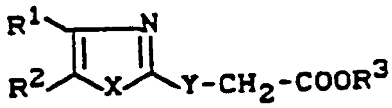

(IV)

dans laquelle

$R^1$, $R^2$, $R^3$, X et Y ont la signification mentionnée ci-dessus,

avec des formamides de formule (Va),

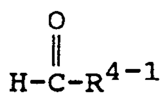

(Va)

dans laquelle

$R^{4-1}$ a la signification mentionnée ci-dessus,

ou avec leurs dérivés de formule (Vb)

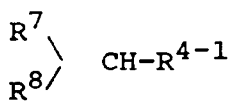

(Vb)

dans laquelle

$R^7$ et $R^8$ représentent chacun, de manière indépendante, un groupe alcool ou un groupe dialkylamino et

$R^{4-1}$ a la signification mentionnée ci-dessus,

éventuellement en présence d'un diluant;ou bien on obtient

(c) des esters acryliques substitués de formule (Ic),

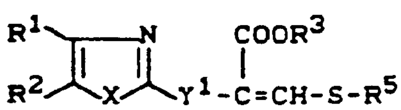

(Ic)

dans laquelle

$Y^1$ représente un atome de soufre ou un radical

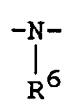

et

$R^1$, $R^2$, $R^3$, $R^5$, $R^6$ et X ont la signification mentionnée ci-dessus,
lorsqu'on fait réagir des dérivés de l'acide oxalique de formule (VI)

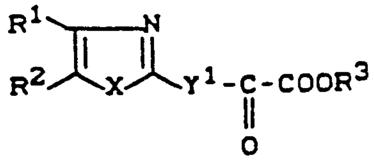

(VI)

dans laquelle
$R^1$, $R^2$, $R^3$, X et $Y^1$ ont la signification mentionnée ci-dessus,
avec des composés organométalliques de formule (VII)

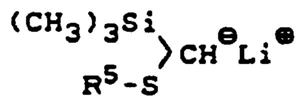

(VII)

dans laquelle
$R^5$ a la signification mentionnée ci-dessus,
éventuellement en présence d'un diluant; ou encore on obtient
(d) des esters acryliques substitués de formule (Id)

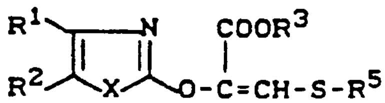

(Id)

dans laquelle
$R^1$, $R^2$, $R^3$, $R^5$ et X ont la signification mentionnée ci-dessus,
lorsqu'on fait réagir des esters acryliques substitués de formule (VIII),

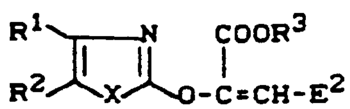

(VIII)

dans laquelle
$E^2$ représente un groupe qui se sépare, attirant les électrons et
$R^1$, $R^2$, $R^3$ et X ont la signification mentionnée ci-dessus,
avec des thiols de formule (IX)

$R^5$-SH (IX)

dans laquelle
$R^5$ a la signification mentionnée ci-dessus,
éventuellement en présence d'un diluant et éventuellement en présence d'un adjuvant réactionnel.

**6.** Agents de lutte contre les parasites, qui se **caractérisent par** le fait qu'ils contiennent au moins un ester acrylique substitué de formule (I) selon la revendication 1 ou 5.

**7.** Utilisation d'esters acryliques substitués de formule (I) selon la revendication 1 ou 5, pour lutter contre les parasites.

**8.** Procédé pour lutter contre les parasites, **caractérisé en ce qu'**on laisse agir des esters acryliques substitués de formule (I) selon la revendication 1 ou 5, sur des parasites et/ou sur leur biotope.

**9.** Procédé pour la préparation d'agents pour la lutte contre les parasites, **caractérisé en ce qu'**on mélange

des esters acryliques substitués de formule (I) selon la revendication 1 ou 5, avec des diluants et/ou des agents tensio-actifs.

**10.** Esters hydroxyacryliques de formule (IIa)

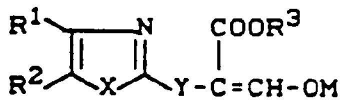

(IIa)

dans laquelle

$R^1$, $R^2$, $R^3$, X, Y ont les significations mentionnées à la revendication 1, et

M représente un atome d'hydrogène ou un cation de métal alcalin, à l'exception du composé ester éthylique de l'acide 2-[4,5-bis(4-méthoxyphényl)thiazol-2-yl-thio] -3-hydroxy-acrylique.

**11.** Procédé pour la préparation d'esters hydroxyacryliques de formule (IIa)

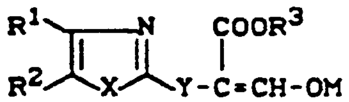

(IIa)

dans laquelle

$R^1$, $R^2$, $R^3$, M, X et Y ont les significations mentionnées à la revendication 10,

**caractérisé en ce qu'**on fait réagir des esters acétiques substitués de formule (IV)

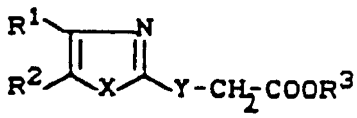

(IV)

dans laquelle

$R^1$, $R^2$, $R^3$, X et Y ont la signification mentionnée ci-dessus, avec des esters d'acide formique de formule (X)

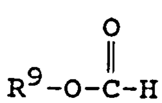

(X)

dans laquelle

$R^9$ représente un groupe alkyle,

éventuellement en présence d'un diluant et éventuellement en présence d'un adjuvant réactionnel basique, à des températures entre -20°C et +50°C.

**12.** Esters acryliques substitués de formule (VIII),

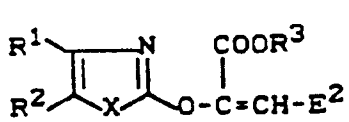

(VIII)

dans laquelle

$R^1$, $R^2$, $R^3$, X ont les significations mentionnées à la revendication 1, et

$E^2$ représente un groupe qui se sépare, attirant les électrons.

**13.** Procédé pour la préparation d'esters acryliques substitués de formule (VIII),

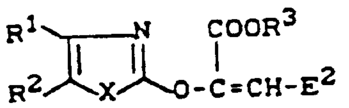

(VIII)

dans laquelle

$R^1$, $R^2$, $R^3$, X et $E^2$ ont les significations mentionnées à la revendication 12,

**caractérisé en ce qu'**on fait réagir des esters hydroxyacryliques de formule (IIb)

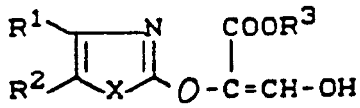

(IIb)

dans laquelle

$R^1$, $R^2$, $R^3$ et X ont la signification indiquée ci-dessus , avec des chlorures d'acides de formule (XIII)

$R^{10}$-Cl (XIII)

dans laquelle

$R^{10}$ représente un radical acyle ou un radical sulfonyle,

éventuellement en présence d'un diluant et éventuellement en présence d'un agent liant ou neutralisant les acides, à des températures entre -20°C et +120°C.

**Revendications pour l'Etat contractant suivant: ES**

1. Procédé pour la préparation d'esters acryliques substitués répondant à la formule générale (I)

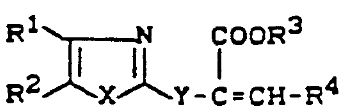

(I)

dans laquelle

$R^1$ et $R^2$ représentent chacun, indépendamment l'un de l'autre, un atome d'hydrogène, un groupe alkyle à chaîne droite ou ramifiée contenant de 1 à 8 atomes de carbone, un groupe alcényle à chaîne droite ou ramifiée contenant de 2 à 8 atomes de carbone, un groupe aralkyle contenant de 1 à 6 atomes de carbone dans la fraction alkyle à chaîne droite ou ramifiée, un groupe aralcényle contenant de 2 à 6 atomes de carbone dans la fraction alcényle à chaîne droite ou ramifiée ou un groupe aryle contenant de 6 à 10 atomes de carbone dans chaque fraction aryle, ces groupes étant chacun éventuellement substitués une ou plusieurs fois de manière identique ou différente dans la fraction aryle, dans lesquels, comme substituants du groupe aryle, entrent chaque fois en ligne de compte : un atome d'halogène, un groupe cyano, un groupe nitro, un groupe alkyle, un groupe alcoxy ou un groupe alkylthio contenant chacun de 1 à 4 atomes de carbone et étant chacun à chaîne droite ou ramifiée, un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée, un groupe alcoxycarbonyle ou un groupe alcoximinoalkyle contenant chacun de 1 à 8 atomes de carbone dans les fractions alkyle individuelles, chacun de ces groupes étant à chaîne droite ou ramifiée, un groupe cycloalkyle contenant de 3 à 7 atomes de carbone, un groupe alcanediyle lié deux fois contenant de 3 à 5 atomes de carbone, un groupe aryle, un groupe aralkyle, un groupe aryloxy ou un groupe aralkyloxy contenant chacun de 6 à 10 atomes de carbone dans la fraction aryle et éventuellement de 1 à 4 atomes de carbone dans la fraction alkyle à chaîne droite ou ramifiée, chacun de ces groupes étant éventuellement substitués une ou plusieurs fois de manière identique ou différente dans la fraction aryle par un atome d'halogène, un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, ou encore un groupe hétéroarylalkyle ou un groupe hétéroaryle contenant chacun de 2 à 9 atomes de carbone et de 1 à 4 hétéro-atomes identiques ou différents dans la fraction hétéroaryle et éventuellement de 1 à 4 atomes de carbone dans la fraction alkyle à chaîne droite ou ramifiée, chacun de ces groupes étant éventuellement substitué une ou plusieurs fois de manière identique ou différente dans la fraction hétéroaryle par un atome d'halogène, un groupe

alkyle, un groupe alcoxy, un groupe alkylthio, un groupe halogénalkyle, un groupe halogénalcoxy, ou un groupe halogénalkylthio contenant chacun de 1 à 4 atomes de carbone et éventuellement de 1 à 9 atomes d'halogène identiques ou différents; en outre, un radical hétéroaryle contenant de 2 à 9 atomes de carbone et de 1 à 4 hétéro-atomes identiques ou différents éventuellement substitué une ou plusieurs fois de manière identique ou différente, dans lequel entrent en ligne de compte comme substituants, les substituants mentionnés ci-dessus pour le groupe aryle,

$R^3$ représente un groupe alkyle à chaîne droite ou ramifiée contenant de 1 à 6 atomes de carbone ou un groupe aralkyle contenant de 1 à 4 atomes de carbone dans la fraction alkyle à chaîne droite ou ramifiée et de 6 à 10 atomes de carbone dans la fraction aryle, ce groupe étant éventuellement substitué une ou plusieurs fois de manière identique ou différente dans la fraction aryle, dans lequel comme substituants du groupe aryle, entrent en ligne de compte ceux mentionnés pour $R^1$ et $R^2$,

$R^4$ représente un groupe dialkylamino contenant chaque fois de 1 à 6 atomes de carbone dans les fractions alkyle individuelles ou bien un radical $-Z-R^5$,

X représente un atome d'oxygène ou un atome de soufre et

Y représente un atome d'oxygène, un atome de soufre ou un radical

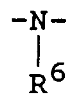

dans lesquels

$R^5$ représente un groupe alkyle à chaîne droite ou ramifiée contenant de 1 à 6 atomes de carbone ou un groupe aralkyle contenant de 1 à 4 atomes de carbone dans la fraction alkyle à chaîne droite ou ramifiée et de 6 à 10 atomes de carbone dans la fraction aryle, ce groupe étant éventuellement substitué une ou plusieurs fois de manière identique ou différente dans la fraction aryle, dans lequel entrent en ligne de compte comme substituants du groupe aryle, ceux mentionnés pour $R^1$ et $R^2$,

$R^6$ représente un atome d'hydrogène, un groupe alkyle à chaîne droite ou ramifiée contenant de 1 à 6 atomes de carbone, un groupe alcanoyle à chaîne droite ou ramifiée contenant de 1 à 6 atomes de carbone dans la fraction alkyle ou un groupe aralkyle contenant de 1 à 6 atomes de carbone dans la fraction alkyle à chaîne droite ou ramifiée, ou encore un groupe aryle contenant chaque fois de 6 à 10 atomes de carbone dans chaque fraction aryle, chacun de ces groupes étant éventuellement substitué une ou plusieurs fois de manière identique ou différente dans la fraction aryle, dans lequel entrent en ligne de compte comme substituants dans la fraction aryle, chaque fois ceux mentionnés pour $R^1$ et $R^2$, et

Z représente un atome d'oxygène ou un atome de soufre,

**caractérisé en ce qu'**on obtient

(a) des esters acryliques substitués de formule (Ia)

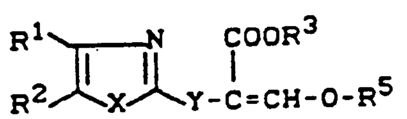 (Ia)

dans laquelle

$R^1$, $R^2$, $R^3$, $R^5$, X et Y ont la signification mentionnée ci-dessus,

lorsqu'on fait réagir des esters hydroxyacryliques ou leurs sels de métaux alcalins de formule (II)

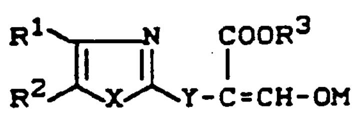 (II)

dans laquelle

M représente un atome d'hydrogène ou un cation de métal alcalin et

$R^1$, $R^2$, $R^3$, X et Y ont la signification mentionnée ci-dessus,

avec des agents d'alkylation de formule (III),

$$R^5-E^1 \quad (III)$$

dans laquelle

$E^1$ représente un groupe qui se sépare, attirant les électrons et
$R^5$ a la signification mentionnée ci-dessus,
éventuellement en présence d'un diluant et éventuellement en présence d'un adjuvant réactionnel; ou on obtient
(b) des esters acryliques substitués de formule (Ib),

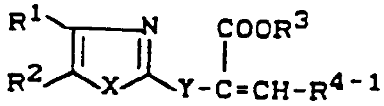 (Ib)

dans laquelle
$R^{4-1}$ représente un groupe dialkylamino et
$R^1$, $R^2$, $R^3$, X et Y ont la signification mentionnée ci-dessus,
lorsqu'on fait réagir des esters acétiques substitués de formule (IV),

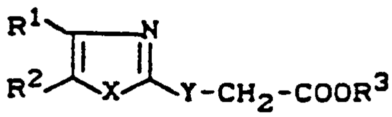 (IV)

dans laquelle
$R^1$, $R^2$, $R^3$, X et Y ont la signification mentionnée ci-dessus,
avec des formamides de formule (Va),

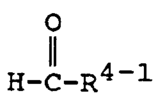 (Va)

dans laquelle
$R^{4-1}$ a la signification mentionnée ci-dessus,
ou avec leurs dérivés de formule (Vb)

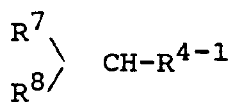 (Vb)

dans laquelle
$R^7$ et $R^8$ représentent chacun, de manière indépendante, un groupe alcoxy ou un groupe dialkylamino et
$R^{4-1}$ a la signification mentionnée ci-dessus,
éventuellement en présence d'un diluant; ou on obtient
(c) des esters acryliques substitués de formule (Ic),

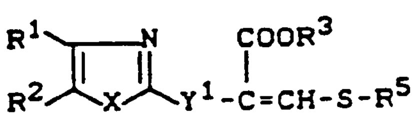 (Ic)

dans laquelle
$Y^1$ représente un atome de soufre ou un radical

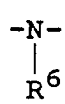

et

$R^1$, $R^2$, $R^3$, $R^5$, $R^6$ et X ont la signification mentionnée ci-dessus,

lorsqu'on fait réagir des dérivés de l'acide oxalique de formule (VI)

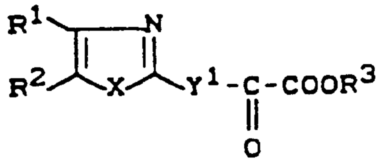

(VI)

dans laquelle

$R^1$, $R^2$, $R^3$, X et $Y^1$ ont la signification mentionnée ci-dessus,

avec des composés organométalliques de formule (VII)

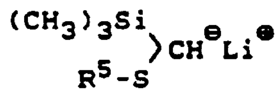

(VII)

dans laquelle

$R^5$ a la signification mentionnée ci-dessus,

éventuellement en présence d'un diluant; ou on obtient

(d) des esters acryliques substitués de formule (Id)

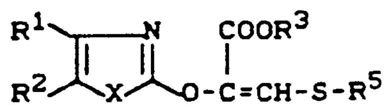

(Id)

dans laquelle

$R^1$, $R^2$, $R^3$, $R^5$ et X ont la signification mentionnée ci-dessus,

lorsqu'on fait réagir des esters acryliques substitués de formule (VIII),

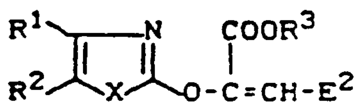

(VIII)

dans laquelle

$E^2$ représente un groupe qui se sépare, attirant les électrons et

$R^1$, $R^2$, $R^3$ et X ont la signification mentionnée ci-dessus,

avec des thiols de formule (IX)

$$R^5\text{-SH} \qquad (IX)$$

dans laquelle

$R^5$ a la signification mentionnée ci-dessus,

éventuellement en présence d'un diluant et éventuellement en présence d'un adjuvant réactionnel.

**2.** Procédé selon la revendication 1, dans lequel, dans la formule (I) :

$R^1$ et $R^2$ représentent chacun, indépendamment l'un de l'autre, un atome d'hydrogène, un groupe méthyle, un groupe éthyle, un groupe n- ou i-propyle, un groupe n-, i-, s- ou t-butyle, un groupe allyle, un groupe n- ou i-butényle, un groupe benzyle, un groupe phényléthyle, un groupe phényléthényle, un groupe phényle, un groupe naphtyle, un groupe pyridyle, un groupe thiényle ou un groupe furyle, chacun de ces groupes étant éventuellement substitué de 1 à 3 fois de manière identique ou différente dans la fraction aryle ou dans la fraction hétéroaryle, dans lesquels entrent chaque fois en ligne de compte comme substituants :

un atome de fluor, un atome de chlore, un atome de brome, un groupe cyano, un groupe nitro, un groupe

méthyle, un groupe éthyle, un groupe n- ou i propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe difluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle, un groupe éthoximinoéthyle, un groupe cyclopentyle, un groupe cyclohexyle, un groupe 1,3-propanediyle, un groupe 1,4-butanediyle ou encore un groupe phényle, un groupe benzyle, un groupe phénoxy ou un groupe benzyloxy, chacun de ces groupes étant éventuellement substitué de 1 à 3 fois de manière identique ou différente dans la fraction phényle par un atome de fluor, un atome de chlore, un atome de brome, un groupe méthyle, un groupe éthyle, un groupe méthoxy, un groupe éthoxy, un groupe méthylthio, un groupe trifluorométhyle, un groupe difluorométhoxy, un groupe trifluorométhoxy et/ou un groupe trifluorométhylthio,

$R^3$ représente un groupe méthyle, un groupe éthyle, un groupe n- ou i-propyle, un groupe n-, i-, s- ou t-butyle ou encore un groupe benzyle éventuellement substitué de 1 à 3 fois de manière identique ou différente, dans lequel entrent en ligne de compte comme substituants, ceux mentionnés pour $R^1$ et $R^2$,

$R^4$ représente un groupe dialkylamino contenant chaque fois de 1 à 4 atomes de carbone dans les fractions alkyle individuelles ou encore un radical -Z-$R^5$,

X représente un atome d'oxygène ou un atome de soufre, et

Y représente un atome d'oxygène, un atome de soufre ou un radical

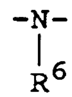

dans lesquels

$R^5$ représente un groupe méthyle, un groupe éthyle, un groupe n- ou i-propyle, un groupe n-, i-, s- ou t-butyle ou encore un groupe benzyle éventuellement substitué de 1 à 3 fois de manière identique ou différente, dans lequel entrent en ligne de compte comme substituants ceux mentionnés pour $R^1$ et $R^2$;

$R^6$ représente un atome d'hydrogène, un groupe méthyle, un groupe éthyle, un groupe n- ou i-propyle, un groupe n-, i-, s- ou t-butyle, un groupe acétyle, un groupe propionyle, un groupe n- ou i-butyryle ou encore un groupe benzyle ou un groupe phényle, chacun de ces groupes étant éventuellement substitué de 1 à 3 fois de manière identique ou différente, dans lequel entrent en ligne de compte comme substituants, ceux mentionnés pour $R^1$ et $R^2$, et

Z représente un atome d'oxygène ou un atome de soufre.

**3.** Procédé selon la revendication 1, dans lequel, dans la formule,

$R^1$ et $R^2$ représentent chacun, indépendamment l'un de l'autre, un atome d'hydrogène, un groupe méthyle, un groupe éthyle, un groupe n- ou i-propyle, ou encore un groupe phényle ou un groupe naphtyle, chacun de ces groupes étant éventuellement substitué de 1 à 3 fois de manière identique ou différente, dans lequel entrent chaque fois en ligne de compte comme substituants :

un atome de fluor, un atome de chlore, un atome de brome, un groupe cyano, un groupe nitro, un groupe méthyle, un groupe éthyle,un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe difluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle, un groupe éthoximinoéthyle, un groupe cyclopentyle, un groupe cyclohexyle, un groupe 1,3-propanediyle, un groupe 1,4-butanediyle ou encore un groupe phényle, un groupe phénoxy, un groupe benzyle ou un groupe benzyloxy, chacun de ces groupes étant éventuellement substitué 1 ou 2 fois de manière identique ou différente par un atome de fluor, un atome de chlore, un atome de brome, un groupe méthyle et/ou un groupe éthyle,

$R^3$ représente un groupe méthyle, un groupe éthyle ou un groupe benzyle,

$R^4$ représente un groupe diméthylamino, un groupe diéthylamino ou un radical -Z-$R^5$,

X représente un atome d'oxygène ou un atome de soufre, et

Y représente un atome d'oxygène, un atome de soufre ou un radical

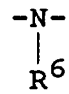

dans lesquels

$R^5$ représente un groupe méthyle, un groupe éthyle, un groupe n- ou i-propyle, ou un groupe benzyle,

$R^6$ représente un atome d'hydrogène, un groupe méthyle, un groupe éthyle, un groupe acétyle, un groupe propionyle ou encore un groupe benzyle ou un groupe phényle, chacun de ces groupes étant éventuellement substitué 1 ou 2 fois de manière identique ou différente par un atome de fluor, un atome de chlore, un atome de brome, un groupe méthyle, un groupe éthyle et/ou un groupe trifluorométhyle, et

Z représente un atome d'oxygène ou un atome de soufre.

**4.** Procédé selon la revendication 1, dans lequel, dans la formule (I),

$R^1$ représente un atome d'hydrogène, un groupe méthyle, un groupe éthyle, un groupe n- ou i-propyle ou encore un groupe phényle éventuellement substitué une ou deux fois de manière identique ou différente, dans lequel entrent en ligne de compte comme substituants :
un atome de fluor, un atome de chlore, un atome de brome, un groupe cyano, un groupe nitro, un groupe méthyle,un groupe éthyle, un groupe méthoxy, un groupe éthoxy, un groupe méthylthio, un groupe trifluorométhyle, un groupe trifluorométhoxy, un groupe trifluorométhylthio, un groupe méthoxycarbonyle, un groupe éthoxycarbonyle, un groupe méthoximinométhyle, un groupe méthoximinoéthyle, un groupe cyclopentyle, un groupe 1,3-propanediyle ou encore un groupe phényle, un groupe phénoxy, un groupe benzyle ou un groupe benzyloxy éventuellement substitués une ou deux fois de manière identique ou différente par un atome de fluor, un atome de chlore, un atome de brome ou un groupe méthyle,

$R^2$ représente un atome d'hydrogène, un groupe méthyle, un groupe éthyle, un groupe n- ou i-propyle,

$R^3$ représente un groupe méthyle ou un groupe éthyle,

$R^4$ représente un groupe méthoxy, un groupe éthoxy, un groupe méthylthio ou un groupe diméthylamino,

X représente un atome d'oxygène ou un atome de soufre, et

Y représente un radical N-méthyle.

**5.** Agents de lutte contre les parasites, qui se **caractérisent par** le fait qu'ils contiennent au moins un ester acrylique substitué de formule (I) selon la revendication 1.

**6.** Utilisation d'esters acryliques substitués de formule (I) selon la revendication 1, pour lutter contre les parasites.

**7.** Procédé pour lutter contre les parasites, **caractérisé en ce qu'**on laisse agir des esters acryliques substitués de formule (I) selon la revendication 1, sur des parasites et/ou sur leur biotope.

**8.** Procédé pour la préparation d'agents pour la lutte contre les parasites, **caractérisé en ce qu'**on mélange des esters acryliques substitués de formule (I) selon la revendication 1, avec des diluants et/ou des agents tensio-actifs.

**9.** Procédé pour la préparation d'esters hydroxyacryliques de formule (IIa)

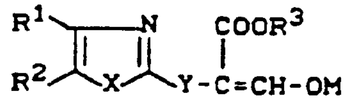

(IIa)

dans laquelle

$R^1$, $R^2$, $R^3$, X, Y ont les significations mentionnées à la revendication 1, et

M représente un atome d'hydrogène ou un cation de métal alcalin, à l'exception du composé ester éthylique de l'acide 2-[4,5-bis(4-méthoxyphényl)thiazol-2-yl-thio] -3-hydroxy-acrylique

**caractérisé en ce qu'**on fait réagir des esters acétiques substitués de formule (IV)

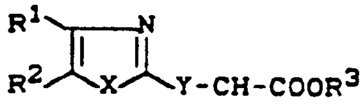

(IV)

dans laquelle

$R^1$, $R^2$, $R^3$, X et Y ont la signification mentionnée ci-dessus, avec des esters d'acide formique de formule (X)

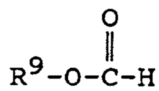

(X)

dans laquelle

$R^9$ représente un groupe alkyle,

éventuellement en présence d'un diluant et éventuellement en présence d'un adjuvant réactionnel basique, à des températures entre -20°C et +50°C.

**10.** Procédé pour la préparation d'esters acryliques substitués de formule (VIII),

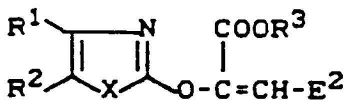

(VIII)

dans laquelle

$E^2$ représente un groupe qui se sépare, attirant les électrons, et

$R^1$, $R^2$, $R^3$, X ont les significations mentionnées à la revendication 1,

**caractérisé en ce qu'**on fait réagir des esters hydroxyacryliques de formule (IIb)

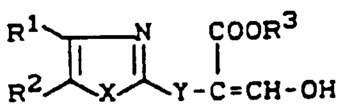

(IIb)

dans laquelle

$R^1$, $R^2$, $R^3$ et X ont la signification indiquée ci-dessus, avec des chlorures d'acides de formule (XIII)

$R^{10}$-Cl (XIII)

dans laquelle

$R^{10}$ représente un radical acyle ou un radical sulfonyle,

éventuellement en présence d'un diluant et éventuellement en présence d'un agent liant ou neutralisant les acides, à des températures entre -20°C et +120°C.